(19) Europäisches Patentamt
European Patent Office
Office européen des brevets

(11) EP 2 376 512 B1

(12) FASCICULE DE BREVET EUROPEEN

(45) Date de publication et mention de la délivrance du brevet:
**26.02.2014 Bulletin 2014/09**

(21) Numéro de dépôt: **09801748.6**

(22) Date de dépôt: **08.12.2009**

(51) Int Cl.:
*C07H 15/24* (2006.01) *C07H 17/04* (2006.01)
*C07H 17/00* (2006.01) *C07H 7/06* (2006.01)
*A61K 31/70* (2006.01) *A61K 31/485* (2006.01)
*A61P 29/00* (2006.01) *A61P 25/04* (2006.01)
*C07D 489/02* (2006.01)

(86) Numéro de dépôt international:
**PCT/FR2009/052448**

(87) Numéro de publication internationale:
**WO 2010/067010 (17.06.2010 Gazette 2010/24)**

# (54) DERIVES DE MORPHINE-6-GLUCURONIDE, LEUR PREPARATION ET LEUR APPLICATION EN THERAPEUTIQUE

MORPHIN-6-GLUCURONIDDERIVATE, VERFAHREN ZU IHRER HERSTELLUNG UND IHRE THERAPEUTISCHE VERWENDUNG

DERIVATIVES OF MORPHINE-6-GLUCURONID, PROCESS FOR THEIR PREPARATION AND THEIR USE IN THERAPY

(84) Etats contractants désignés:
**AT BE BG CH CY CZ DE DK EE ES FI FR GB GR HR HU IE IS IT LI LT LU LV MC MK MT NL NO PL PT RO SE SI SK SM TR**
Etats d'extension désignés:
**AL BA RS**

(30) Priorité: **10.12.2008 FR 0806949**

(43) Date de publication de la demande:
**19.10.2011 Bulletin 2011/42**

(73) Titulaire: **SANOFI**
**75008 Paris (FR)**

(72) Inventeurs:
- **DLUBALA, Alain**
  **F-75013 Paris (FR)**
- **RIPOCHE, Isabelle**
  **F-63174 Aubière Cedex (FR)**
- **TRECANT, Claire**
  **F-67000 Strasbourg (FR)**

(74) Mandataire: **Veinante, Aude et al**
**Sanofi**
**Département Brevets**
**54, rue La Boétie**
**75008 Paris (FR)**

(56) Documents cités:
**WO-A-98/46618 FR-A- 2 864 082**

- **CHENG GANG ET AL: "syn additions to 4alpha-epoxypyranosides: synthesis of L-idopyranosides.", ORGANIC LETTERS 8 NOV 2007, vol. 9, no. 23, 8 novembre 2007 (2007-11-08), pages 4849-4852, XP002544745, ISSN: 1523-7060**
- **MCMILLAN K G ET AL: "Synthesis, structure and reactivity of 5-pyranosyl-1,3,4-oxathiazol-2-on", CARBOHYDRATE RESEARCH, ELSEVIER SCIENTIFIC PUBLISHING COMPANY. AMSTERDAM, NL, vol. 341, no. 1, 16 janvier 2006 (2006-01-16), pages 41-48, XP025010249, ISSN: 0008-6215 [extrait le 2006-01-16]**
- **DANISHEFSKY S J ET AL: "A STEREOSELECTIVE TOTALLY SYNTHETIC ROUTE TO METHYL ALPHA-PERACETYLHIKOSAMINIDE", JOURNAL OF THE AMERICAN CHEMICAL SOCIETY, vol. 111, no. 6, 1989, pages 2193-2204, XP002544746, ISSN: 0002-7863**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

**Description**

**DOMAINE DE L'INVENTION**

La présente invention se rapporte à des dérivés de morphine-6-glucuronide, à leur préparation et à leur utilisation pour le traitement et la prévention de la douleur.

Les dérivés de morphine-6-glucuronide et les dérivés de morphine-6-glucuronamide sont décrits dans les demandes de brevets WO 98/46618 et FR 2 864 082 en tant qu'analgésiques.

La présente invention a pour objet les composés répondant à la formule (I)

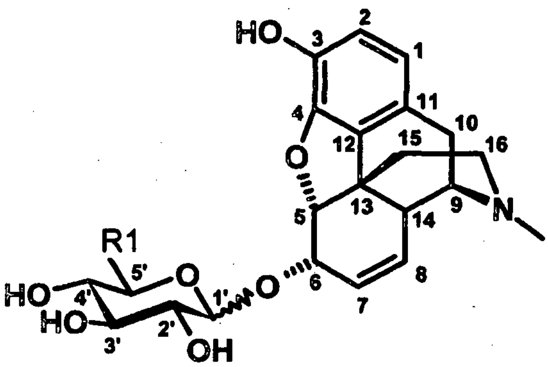

(I)

dans laquelle :

R1 est un groupe hétéroaromatique à 5 chaînons éventuellement substitué par un ou plusieurs substituants choisis parmi les atomes d'halogène et les groupes ($C_1$-$C_4$)alkyle,, hydroxyle, oxo, halo($C_1$-$C_4$)alkyle, halo($C_1$-$C_4$)alkyloxy, ($C_1$-$C_4$)alkyloxy, aryl($C_1$-$C_4$)alkyle et aryle, ledit groupe aryle étant éventuellement substitué par un ou plusieurs groupes choisis parmi les groupes ($C_1$-$C_4$)alkyle, halo($C_1$-$C_4$)alkyle, hydroxyle et ($C_1$-$C_4$)alkyloxy, à l'état de base ou de sel d'addition à un acide ainsi qu'à l'état d'hydrate ou de solvate.

Les composés de formule (I) peuvent comporter un ou plusieurs atomes de carbone asymétrique. Ils peuvent donc exister sous forme d'énantiomères ou de diastéréoisomères. Ces énantiomères et diastéréoisomères, ainsi que leurs mélanges, y compris les mélanges racémiques, font partie de l'invention.

Les composés de formule (I) comportent un carbone anomérique. Ils peuvent exister sous la forme d'anomères $\alpha$ ou $\beta$. Les anomères $\alpha$, $\beta$ et leur mélange font partie de l'invention.

Les composés de formule (I) peuvent exister à l'état de bases ou de sels d'addition à des acides. De tels sels d'addition font partie de l'invention.

Ces sels peuvent être préparés avec des acides pharmaceutiquement acceptables, mais les sels d'autres acides utiles par exemple pour la purification ou l'isolement des composés de formule (I) font également partie de l'invention.

Les composés de formule (I) peuvent également exister sous forme d'hydrates ou de solvates, à savoir sous forme d'associations ou de combinaisons avec une ou plusieurs molécules d'eau ou avec un solvant. De tels hydrates et solvates font également partie de l'invention.

Dans le cadre de la présente invention, on entend par :

- un atome d'halogène : un atome de fluor, de chlore, de brome ou d'iode ;
- un groupe ($C_1$-$C_4$)alkyle : un groupe aliphatique saturé, linéaire ou ramifié, substitué ou non substitué, ayant entre 1 et 4 atomes de carbone ; à titre d'exemples, on peut citer les groupes méthyle, éthyle, propyle, isopropyle, butyle, isobutyle et tertbutyle ;
- un groupe halo($C_1$-$C_4$)alkyle : un groupe alkyle dont un ou plusieurs atomes d'hydrogène ont été substitués par un atome d'halogène tel que défini ci-dessus ; à titre d'exemples de groupes groupe halo($C_1$-$C_4$)alkyle, on peut citer en particulier les groupes fluorométhyle, difluorométhyle, trifluorométhyle, chlorométhyle, dichlorométhyle, trichlorométhyle, fluoroéthyle, difluoroéthyle, trifluoroéthyle, chloroéthyle, dichloroéthyle et trichloroéthyle ;
- un groupe hydroxyle : un groupe -OH ;
- un groupe oxo : groupe = O
- un groupe ($C_1$-$C_4$)alkyloxy : un groupe -O-($C_1$-$C_4$)alkyle où le groupe ($C_1$-$C_4$)alkyle est tel que précédemment défini ;

à titre d'exemples, on peut citer les groupes méthoxy, éthoxy, propoxy et butyloxy ;

- un groupe halo($C_1$-$C_4$)alkyloxy : un groupe ($C_1$-$C_4$)alkyloxy dont un ou plusieurs atomes d'hydrogène ont été substitués par un atome d'halogène tel que défini ci-dessus ; à titre d'exemples, on peut citer les groupes $-OCF_3$, $-OCHF_2$ et $-OCCl_3$ ;
- un groupe aryle : un groupe aromatique cyclique, substitué ou non substitué, comprenant entre 5 et 14 atomes de carbone ; à titre d'exemples de groupes aryles non substitués, on peut citer les groupes phényle et naphtyle; à titre d'exemples de groupes aryles substitués, on peut citer les groupes ($C_1$-$C_4$)alkyloxyphényle tels que les groupes méthoxyphényle, éthoxyphényle, propoxyphényle et butyloxyphényle ;
- un groupe aryl($C_1$-$C_4$)alkyle : un groupe alkyle dont un ou plusieurs atomes d'hydrogène ont été remplacés par un groupe aryle ; à titre d'exemples, on peut citer le groupe benzyle ;
- un groupe hétérocycle hétéroaromatique à 5 chaînons: un groupe aromatique cyclique ayant de 1 à 4 atomes de carbone et comprenant un ou plusieurs hétéroatomes, tels que l'azote, l'oxygène ou le soufre ; à titre d'exemples de groupes hétérocycles hétéroaromatiques à 5 chaînons, on peut citer les groupements pyrrolyle, furanyle, thiophényle, pyrazolyle, imidazolyle, triazolyle, tétrazolyle, oxazolyle, isoxazolyle, oxadiazolyle, thiazolyle, isothiazolyle et thiadiazolyle.

Parmi les composés de formule **(I)** objets de l'invention, un premier groupe de composés présentent une ou plusieurs des caractéristiques suivantes :

- le groupe hétérocycle hétéroaromatique est choisi parmi les groupes pyrrole, furane, thiophène, imidazole, triazole, tétrazole, oxazole, isoxazole, oxadiazole, thiazole, isothiazole et thiadiazole, et
- lorsque le groupe hétérocycle hétéroaromatique est substitué par un ou plusieurs groupes, ledit groupe est choisi parmi les groupes méthyle, éthyle, propyle, isopropyle, butyle, isobutyle, tertbutyle, fluorométhyle, difluorométhyle, trifluorométhyle, chlorométhyle, dichlorométhyle, trichlorométhyle, fluoroéthyle, difluoroéthyle, trifluoroéthyle, chloroéthyle, dichloroéthyle, trichloroéthyle, méthoxyphényle, éthoxyphényle, propoxyphényle et butyloxyphényle.

Parmi les composés cités précédemment, on peut citer en particulier les composés de formule **(I)** pour lesquels :

- le groupe hétérocycle hétéroaromatique est choisi parmi les groupes tétrazole, triazole, en particulier le 1,2,4 triazole, et oxadiazole, en particulier le 1,3,4 oxadiazole, et
- lorsque le groupe hétérocycle hétéroaromatique est substitué par au moins un groupe, ledit groupe est choisi parmi les groupes méthyle, trifluoroéthyle et p-méthoxyphényle.

Parmi les composés de formule (I) objets de l'invention, on peut notamment citer les composés suivants :

- le morphin-6-yl 5-*C*-(tétrazol-5-yl)-$\alpha/\beta$-D-xylopyranoside (1/1),
- le morphin-6-yl 5-*C*-(tétrazol-5-yl)-$\beta$-D-xylopyranoside
- le morphin-6-yl 5-*C*-(2-méthyl-1,3,4-oxadiazol-5-yl)-$\alpha/\beta$-D-xylopyranoside (2/3)
- le morphin-6-yl 5-*C*-(2-méthyl-1,3,4-oxadiazol-5-yl)-$\alpha$-D-xylopyranoside
- le morphin-6-yl 5-*C*-(2-méthyl-1,3,4-oxadiazol-5-yl)-$\beta$-D-xylopyranoside et
- le morphin-6-yl 5-*C*-[5-(4-méthoxyphényl)-4-(2,2,2-trifluoroéthyl)-4*H*-1,2,4-triazol-3-yl]-$\beta$-D-xylopyranoside.

**PROCEDE DE PREPARATION**

Dans ce qui suit, on entend par groupe protecteur PG un groupe qui permet, d'une part, de protéger une fonction réactive telle qu'un hydroxy ou une amine pendant une synthèse et, d'autre part, de régénérer la fonction réactive intacte en fin de synthèse. Des exemples de groupes protecteurs ainsi que des méthodes de protection et de déprotection sont données dans « Protective Groups in Organic Synthesis », Green et al., 2nd Edition (John Wiley & Sons, Inc., New York).

On entend par groupe partant LG, dans ce qui suit, un groupe pouvant être facilement clivé d'une molécule par rupture d'une liaison hétérolytique, avec départ d'une paire électronique. Ce groupe peut ainsi être remplacé facilement par un autre groupe lors d'une réaction de substitution, par exemple. De tels groupes partants sont, par exemple, les halogènes ou un groupe hydroxy activé tel qu'un méthanesulfonate, benzènesulfonate, p-toluènesulfonate, triflate, acétate, etc. Des exemples de groupes partants ainsi que des références pour leur préparation sont donnés dans « Advances in Organic Chemistry », J. March, 3rd Edition, Wiley Interscience, p. 310-316.

Conformément à l'invention, on peut préparer les composés de formule générale (I) selon le procédé qui suit illustré par le schéma 1.

## Schéma 1

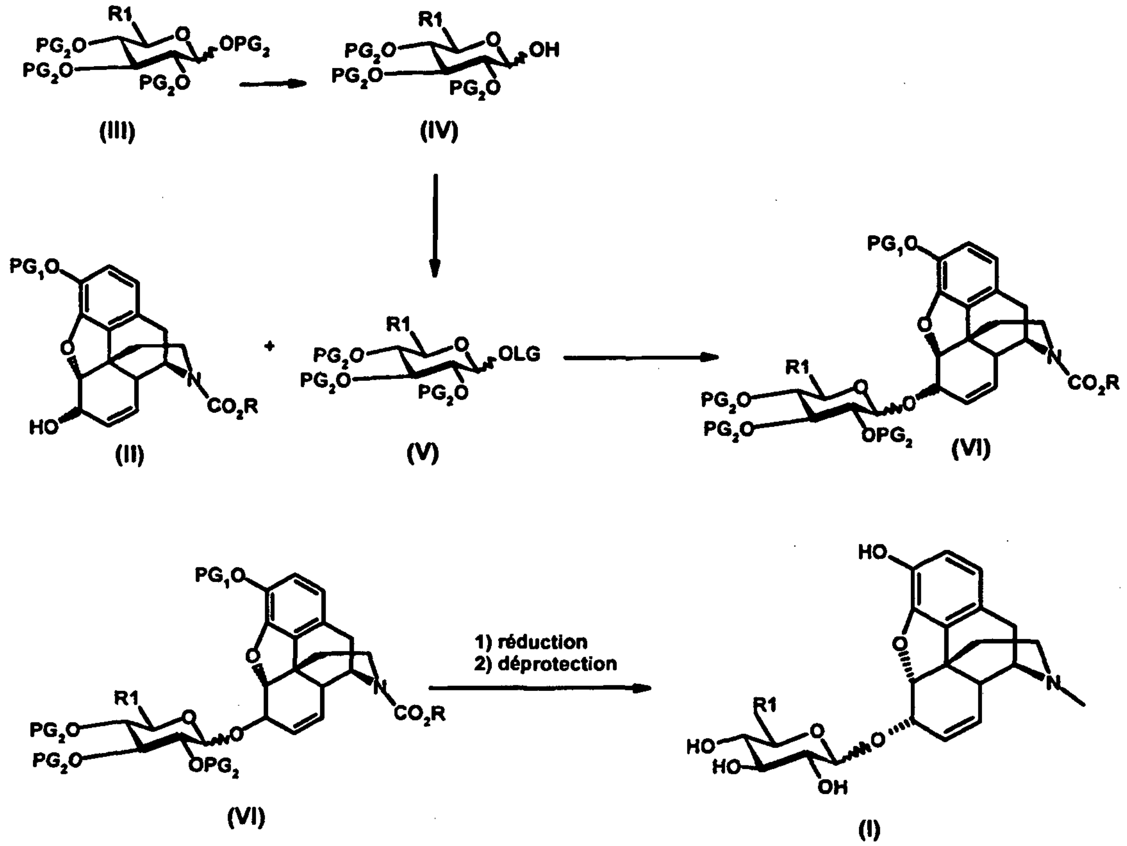


(III) (IV) (II) (V) (VI) (VI) (I)

Dans un première étape, un composé de formule générale (II), dans laquelle $PG_1$ représente un groupe protecteur tel qu'un groupe pivaloyle et R représente un groupe $(C_1\text{-}C_4)$-alkyle, par exemple un méthyle ou un éthyle, peut être couplé à un composé de formule générale (V), dans laquelle R1 est tel que défini dans la formule générale (I), $PG_2$ est un groupe protecteur tel qu'un groupe benzoyle et LG est un groupe activateur tel qu'un groupe trichloroacétimidate $-CNHCCl_3$, pour obtenir un composé de formule générale (VI).

La réaction de couplage peut par exemple s'effectuer en présence d'un acide de Lewis, tel que le trifluorométhanesulfonyl de triméthylsilane (TMSOTf), dans un solvant tel que le dichlorométhane, à une température comprise entre 0°C et 25°C.

Dans certains cas, le composé de formule générale (V) pourra être protégé préalablement à la réaction de couplage. Par exemple lorsque R1 représente un groupe tétrazolyle, celui-ci pourra être préalablement protégé par un groupe protecteur tel qu'un groupe 4-méthoxybenzyle. Postérieurement à la réaction de couplage, ce groupe protecteur pourra être clivé, par exemple dans le cas d'un groupe tétrazolyle portant un groupe 4-méthoxybenzyle, en présence d'acide trifluoroacétique (TFA) à la température de reflux.

Le composé de formule générale (II) peut être préparé par exemple selon la méthode décrite dans Portoghese and coll. J. Med. Chem. 1972, 15, 208-210.

Le composé de formule générale (V) peut être obtenu par activation de la fonction hydroxyle du composé de formule générale (IV), dans laquelle R1 et PG2 sont tels que définis ci-dessus. Dans le cas où le groupe LG est un groupe partant tel qu'un groupe trichloroacétimidate $-CNHCCl_3$, la réaction peut se faire en présence de trichloroacétonitrile et d'une base forte telle que le 1,8-diazabicyclo[5.4.0]undec-7-ène, dans un solvant tel que le dichlorométhane.

Le composé de formule générale (IV) peut être préalablement obtenu par déprotection anomérique du composé de formule générale (III) dans laquelle R1 et PG2 sont tels que définis ci-dessus. Dans le cas où PG2 représente un groupe benzoyle, la déprotection du groupe hydroxyle peut se faire en présence d'acétate d'hydrazine (NH2NH2, CH3COOH).

Dans une seconde étape, le composé de formule générale (VI) est réduit et déprotégé simultanément, par exemple en présence d'hydrure de lithium et d'aluminium, dans un solvant tel que le tétrahydrofurane, à la température de reflux du milieu réactionnel, puis isolé en présence d'un acide minéral tel que l'acide chlorhydrique. On obtient ainsi le composé de formule générale (I).

Lorsque R1 représente un groupe 2-méthyl-1,3,4-oxadiazol-5-yle, le composé de formule générale (I) peut être

obtenu à partir du composé de formule générale (I) correspondant pour lequel R1 représente un groupe tétrazolyle, ce dernier étant préparé selon la méthode illustrée dans le schéma 2.

**Schéma 2**

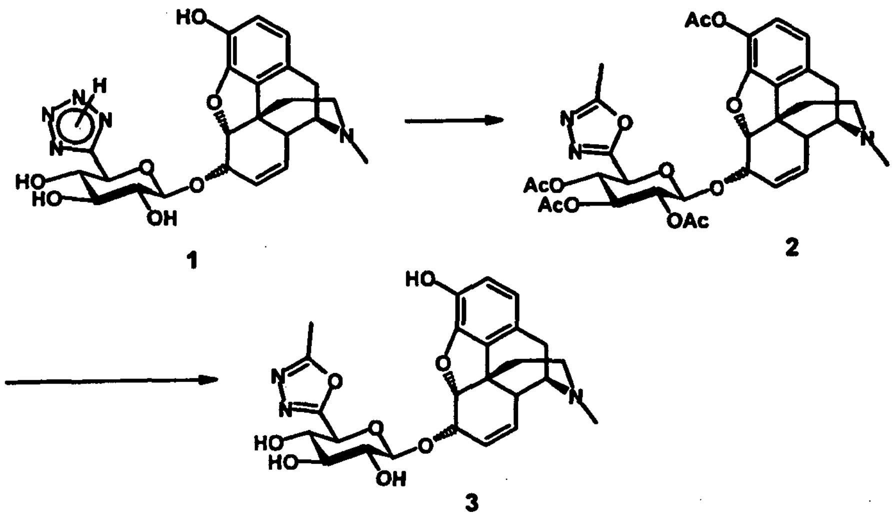

1

2

3

Selon le schéma 2 et dans une première étape, le morphin-6-yl 5-*C*-(tétrazol-5-yl)-β-D-xylopyranoside (1) peut réagir avec un chlorure d'acyle ou un anhydride d'acide, tel que l'anhydride acétique, selon un réarrangement thermique de Huisgen, pour donner le 3-*O*-acétylmorphin-6-yl 2,3,4-tri-*O*-acétyl-5-*C*-(2-méthyl-1,3,4-oxadiazol-5-yl)-β-D-xylopyranoside (2). Dans une seconde étape, les groupements hydroxyles sont déprotégés, par exemple en présence de méthylate de sodium pour obtenir le morphin-6-yl 5-*C*-(2-méthyl-1,3,4-oxadiazol-5-yl)-β-D-xylopyranoside (3).

Dans les schémas 1 et 2, les composés de départ et les réactifs, quand leur mode de préparation n'est pas décrit, sont disponibles dans le commerce ou décrits dans la littérature, ou bien peuvent être préparés selon des méthodes qui y sont décrites ou qui sont connues de l'Homme du métier.

**Intermédiaires de synthèse**

L'invention, selon un autre de ses aspects, a également pour objet les composés de formules générales (III), (IV) et (V). Ces composés sont utiles comme intermédiaires de synthèse des composés de formule générale (I).

L'invention a plus particulièrement pour objet les composés de formule (IIIa), (IIIb), (IIIc), (IVb), (IVc), (Vb) et (Vc).

Les composés (IIIb), (IVb) et (Vb) existent sous forme de mélange des deux isomères de position du groupe paraméthoxybenzyle (PMB).

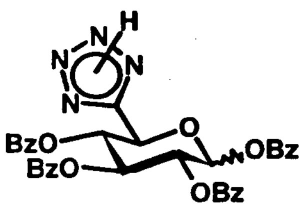

**(IIIa)**

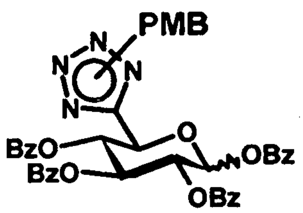

**(IIIb)**

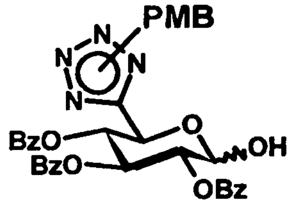

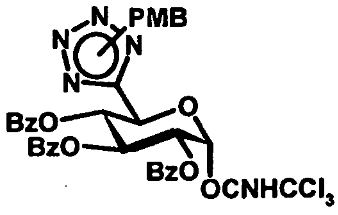

(IVb) (Vb)

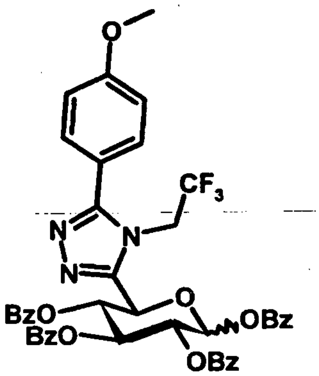

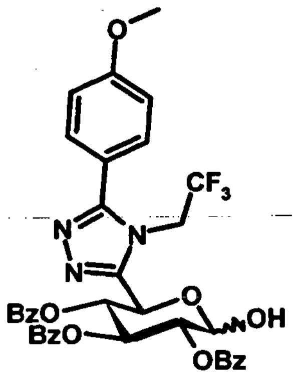

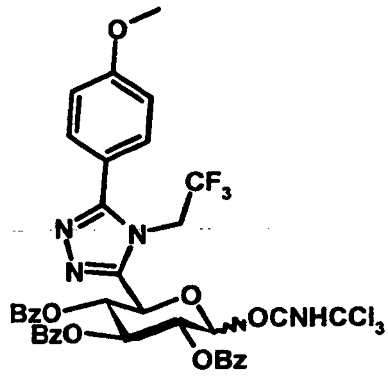

(IIIc) (IVc) (Vc)

Les composés de formule (IIIa), (IIIb), (IVb) et (Vb) peuvent être obtenus selon la méthode décrite dans le schéma 3.

Schéma 3

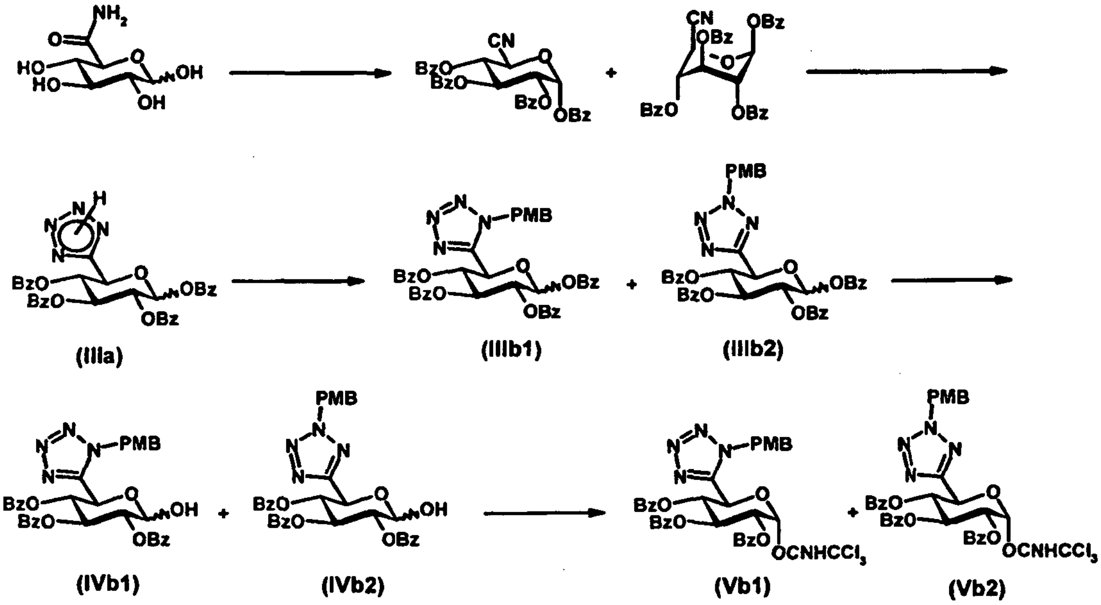

(IIIa) (IIIb1) (IIIb2)

(IVb1) (IVb2) (Vb1) (Vb2)

Dans le schéma 3, le composé de formule (IIIa) protégé, par exemple par des groupements benzoate, peut être obtenu par protection, déshydratation, puis tétrazolylation de la D-glucuronamide. Une méthode de tétrazolylation consiste à faire réagir la fonction nitrile au reflux du toluène en présence d'azoture de triméthylsilyle ($TMSN_3$) et d'oxyde de bis-(tributylétain) $(Bu_3Sn)_2O$. Concernant la réaction de protection du D-glucuronamide, une adaptation de la méthode décrite dans Carbohydrate Research 2006, 341, 1, 41-48 peut être utilisée.

Le composé de formule (IIIb), consistant en un mélange de composés (IIIb1) et (IIIb2), peut être obtenu à partir du composé de formule (IIIa) par protection de l'amine, par exemple par un para-méthoxybenzyle.

La déprotection sélective de la position anomérique du composé de formule (IIIb) permet d'obtenir le composé de formule (IVb), consistant en un mélange de composés (IVb1) et (IVb2). Dans le cas d'un groupement protecteur de type benzoate, cette déprotection peut se faire en présence d'acétate d'hydrazine.

La fonction hydroxyle libre du composé de formule (IVb) peut être transformée en imidate pour générer le composé de formule (Vb), consistant en un mélange de composés (Vb1) et (Vb2). La transformation de la fonction hydroxyle en fonction imidate peut par exemple être effectuée en présence de trichloroacétonitrile et de DBU (1,8-diazabicyclo[5.4.0]undec-7-ène) selon une adaptation de la méthode décrite dans Tetrahedron 2000, 56, 7591-7594.

Les composés de formule (IIIc), (IVc) et (Vc) peuvent être obtenus selon la méthode décrite dans le schéma 4.

## Schéma 4

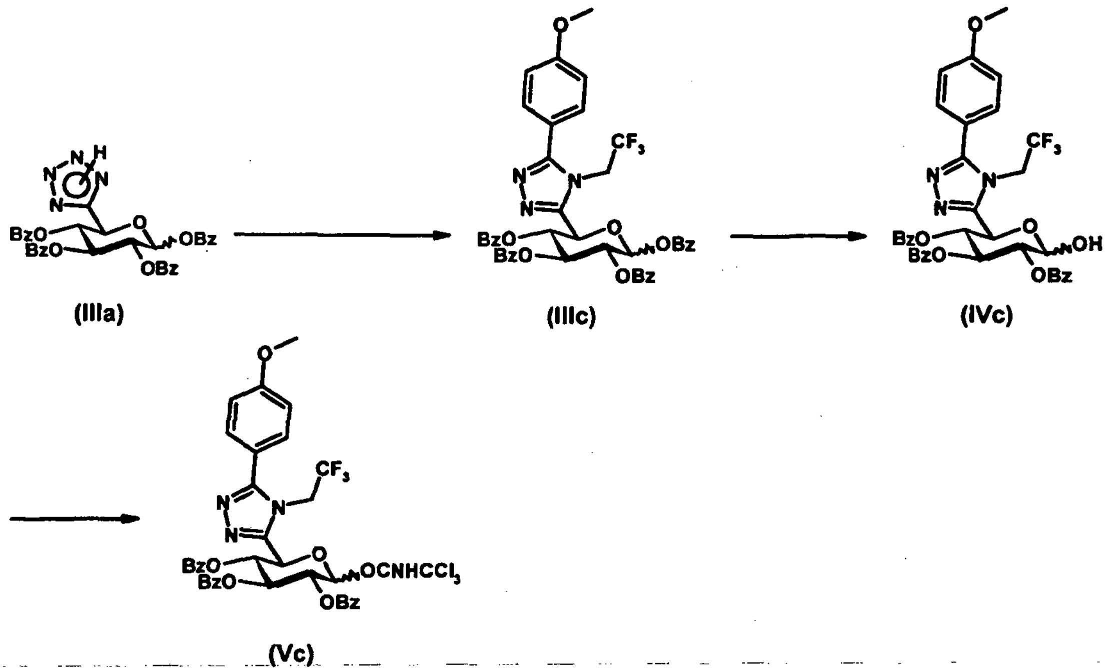

(IIIa) (IIIc) (IVc) (Vc)

Selon le schéma 4, le composé de formule (IIIa) décrit ci-dessus peut subir un réarrangement thermique de Huisgen en présence d'un chlorure d'imidoyle, par exemple le chlorure de 2,2,2-trifluoro-N-(4-méthoxybenzyl)acétimidoyle, pour donner le composé de formule (IIIc).

La déprotection sélective de la position anomérique du composé de formule (IIIc) permet d'obtenir le composé de formule (IVc). Dans le cas d'un groupement protecteur de type benzoate, cette déprotection peut se faire en présence d'acétate d'hydrazine.

La fonction hydroxyle libre du composé de formule (IVc) peut être transformée en imidate pour générer le composé de formule (Vc). La transformation de la fonction hydroxyle en fonction imidate peut par exemple être effectuée en présence de trichloroacétonitrile et de DBU (1,8-diazabicyclo[5.4.0]undec-7-ène) selon une adaptation de la méthode décrite dans Tetrahedron 2000, 56, 7591-7594.

L'invention est illustrée de manière non limitative par les exemples ci-dessous.

Les exemples suivants décrivent la préparation de certains composés conformes à l'invention. Ces exemples ne sont pas limitatifs et ne font qu'illustrer la présente invention. Les numéros des composés exemplifiés renvoient à ceux donnés dans le tableau ci-après, qui illustre les structures chimiques et les propriétés physiques de quelques composés selon l'invention.

## EXEMPLES

**<u>Exemple 1</u> : trifluoroacétate de morphin-6-yl 5-*C*-(tétrazol-5-yl)-β-D-xylopyranoside (composé n°2)**

### 1.1. 1,2,3,4-tétra-*O*-benzoyl-α/β-D-glucurononitrile

A une suspension de D-glucuronamide (25,0 g, 0,129 mol) dans la pyridine (100 mL) à température ambiante, est ajoutée en 30 min une solution de chlorure de benzoyle (102 mL, 0,878 mol) dans du dichlorométhane (90 mL). Le milieu réactionnel est agité une nuit à température ambiante puis du dichlorométhane (200 mL) et de l'eau (200 mL) sont ajoutés. La phase organique est lavée avec une solution d'acide chlorhydrique 1 N (200 mL), une solution saturée d'hydrogénocarbonate de sodium (3 x 200 mL) et une solution saturée de chlorure de sodium (200 mL). La phase organique est séchée sur sulfate de sodium et le solvant éliminé sous pression réduite. Le résidu (huile jaune) est trituré dans l'éthanol (200 mL) pour donner un mélange d'anomères (43,4 g, 57%) sous forme de cristaux de couleur jaune pâle. Le spectre de RMN du proton dans le deutériochloroforme, $CDCl_3$, montre un rapport α:β de 2:1.

Point de fusion : 209-212°C.

RMN $^1H$ (400 MHz, $CDCl_3$) : δ 8,10-7,30 (m, 20Hα+20Hβ, H-*aro*), 6,88 (d, 1Hα, *J* 3,5 Hz, H-1α), 6,57 (d, 1Hβ, *J* 3,0 Hz, H-1β), 6,21 (t, 1Hα, *J* 9,5 Hz, H-3α), 5,93 (t, 1Hα, *J* 9,5 Hz, H-4α), 5,84 (t, 1Hβ, *J* 4,0 Hz, H-3β), 5,71-5,65 (m, 1Hα+1Hβ, H-2α, H-4β), 5,64 (m, 1Hβ, H-2β), 5,16 (d, 1Hβ, *J* 4,0 Hz, H-5β), 5,11 (d, 1Hα, *J* 9,5 Hz, H-5α).

RMN $^{13}C$ (100 MHz, $CDCl_3$) δ 165,5, 165,1, 164,8, 164,6, 164,3, 163,8 (C=O), 134,4-128,0 (C-*aro*), 115,3 (C-6β), 114,1 (C-6α), 90,9 (C-1β), 89,4 (C-1α), 69,3, 69,2, 69,0 (C-2α, C-3α, C-4α), 67,4 (C-4β), 66,7, 66,5 (C-2β, C-3β), 61,9 (C-5α), 60,8 (C-5β).

Masse calculée pour $C_{34}H_{25}NO_9Na$ $[M+Na]^+$ 614,1427, trouvée 614,1422.

### 1.2. 1,2,3,4-tétra-*O*-benzoyl-5-*C*-(tétrazol-5-yl)-α/β-D-xylopyranose

A une solution de 1,2,3,4-tétra-*O*-benzoyl-α/β-D-glucurononitrile précédemment préparé (43,0 g, 72,8 mmol) dans du toluène (500 mL) sont ajoutés de l'oxyde de bis-(tributylétain) (3,70 mL, 7,26 mmol) et de l'azidure de triméthylsilyle (28,7 mL, 216 mmol). Le milieu réactionnel est agité une nuit à reflux. Le solvant est éliminé sous pression réduite et le résidu est purifié par chromatographie sur gel de silice (cyclohexane-acétate d'éthyle 1:1 à 0:1) pour donner le 1,2,3,4-tétra-*O*-benzoyl-5-*C*-(tétrazol-5-yl)-α/β-D-xylopyranose (27,0 g, 59%) sous forme de cristaux bruns. Le spectre de RMN du proton dans $CDCl_3$ montre un rapport α:β de 2:1.

Point de fusion 144-147°C.

RMN $^1H$ (400 MHz, $CDCl_3$) : δ 8,19-7,28 (m, 20Hα+20Hβ, H-*aro*), 7,06 (d, 1Hα, *J* 3,5 Hz, H-1α), 6,50-6,44 (m, 1 Hβ+1Hα, H-1β, H-3α), 6,21 (t, 1 Hβ, *J* 9,0 Hz, H-3β), 6,13-6,01 (m, 2Hβ+1Hα, H-4β, H-4α, H-2β), 5,90-5,85 (m, 2Hα, H-2α, H-5α), 5,66 (d, 1 Hβ, *J* 9,0 Hz, H-5β).

RMN $^{13}C$ (100 MHz, $CDCl_3$) δ 165,79, 165,2, 164,7 (C=O, C=N), 134,4-128,1 (C-*aro*), 93,0 (C-1β), 89,9 (C-1α), 72,0, 70,5, 70,4, 70,3, 69,5 (C-2α, C-2β, C-3α, C-3β C-4α, C-4β), 68,9 (C-5β), 67,0 (C-5α).

Masse calculée pour $C_{34}H_{26}N_4O_9Na$ $[M+Na]^+$ 657,1597, trouvée 657,1595.

### 1.3. 1,2,3,4-tétra-*O*-benzoyl-5-*C*-[2-(4-méthoxybenzyl)-2*H*-tétrazol-5-yl]-α/β-D-xylopyranose (IIIb2) et 1,2,3,4-tétra-*O*-benzoyl-5-*C*-[1-(4-méthoxybenzyl)-1*H*-tétrazol-5-yl]-α/β-D-xylopyranose (IIIb1)

A une solution de 1,2,3,4-tétra-*O*-benzoyl-5-*C*-(tétrazol-5-yl)-α/β-D-xylopyranose (21,0 g, 33,12 mmol) dans du tétrahydrofurane (210 mL) sont ajoutés de la triéthylamine (5,5 mL, 39,46 mmol) et du chlorure de 4-méthoxybenzyle (5,0 mL, 36,71 mmol). Le milieu réactionnel est agité une nuit à reflux. Le solvant est éliminé sous pression réduite et le résidu purifié par chromatographie sur gel de silice (cyclohexane-acétate d'éthyle 4:1) pour donner un mélange d'isomères (18,5 g, 73%) sous forme de cristaux de couleur jaune pâle. Le spectre de RMN du proton dans $CDCl_3$ montre un rapport (IIIb1):(IIIb2) de 2:1 avec pour (IIIb1) un rapport α:β de 2:1 et pour (IIIb2) un rapport α:β de 2:1.

RMN $^1H$ (400 MHz, $CDCl_3$) pour les anomères α : δ 8,21-7,29 (m, 20Ha+20Hb, H-*aro*), 7,23 (d, 2Hb, *J* 8,5 Hz, H-*aroPMB*b), 7,17 (d, 2Ha, *J* 8,5 Hz, H-*aroPMB*a), 6,98 (d, 1Ha, *J* 3,5 Hz, H-1a), 6,96 (d, 1Hb, *J* 3,5 Hz, H-1 b), 6,86 (d, 2Hb, *J* 8,5 Hz, H-*aroPMB*b), 6,75 (d, 2Ha, *J* 8,5 Hz, H-*aroPMB*a), 6,42 (t, 1Ha, *J* 10,0 Hz, H-3a), 6,33 (t, 1Hb, *J* 10,0 Hz, H-3b), 6,13 (t, 1Ha, *J* 10,0 Hz, H-4a), 5,84 (dd, 1 Ha, *J* 3,5 Hz, *J* 10,0 Hz, H-2a), 5,76-5,63 (m, 3Ha+5Hb, $CH_2PhOCH_3$a, $CH_2PhOCH_3$b, H-5a, H-2b, H-4b, H-5b), 3,79 (s, 3Hb, $OCH_3$b), 3,75 (s, 3Ha, $OCH_3$a).

RMN $^{13}C$ (100 MHz, $CDCl_3$) pour les anomères α : δ 165,8, 165,2, 164,5, 164,3, 164,2, 162,1 (C=O, C=N), 134,4-124,7 (C-*aro*), 114,5 (C-*aroPMB*a), 114,3 (C-*aroPMB*b), 90,0 (C-1a), 89,7 (C-1b), 71,1 (C-4a), 70,4, 70,2, (C-2a, C-3a), 69,9, 69,6, 69,5 (C-2b, C-3b, C-4b), 67,0 (C-5a), 66,0 (C-5b), 56,7 ($CH_2PhOCH_3$a), 55,3 ($OCH_3$b), 55,2 ($OCH_3$a), 52,1 ($CH_2PhOCH_3$b).

Masse calculée pour $C_{42}H_{34}N_4O_{10}Na$ $[M+Na]^+$ 777,2173, trouvée 777,2181.

**1.4. 2,3,4-tri-*O*-benzoyl-5-*C*-[2-(4-(méthoxybenzyl)-2*H*-tétrazol-5-yl]-α/β-D-xylopyranose (IVb2) et 2,3,4-tri-*O*-benzoyl-5-*C*-[1-(4-(méthoxybenzyl)-1*H*-tétrazol-5-yl]-α/β-D-xylopyranose (IVb1))**

A une solution du mélange obtenu à l'étape 1.3 (13 ,4 g, 17,77 mmol) dans du *N,N*-diméthylformamide (100 mL) à 0°C, est ajouté de l'acétate d'hydrazine (2,45 g, 26,60 mmol) par petites portions en 15 min. Le milieu réactionnel est agité 1 h à 0°C puis 4 h à température ambiante. Le solvant est éliminé sous pression réduite et le résidu purifié par chromatographie sur gel de silice (cyclohexane-acétate d'éthyle 7:3) pour donner un mélange d'isomères du produit attendu (8,0 g, 70%) sous forme de cristaux jaune. Le spectre de RMN du proton dans $CDCl_3$ montre un rapport (IVb2):(IVb1) de 2:1 avec pour (IVb2) un rapport α:β de 5:1 et pour (IVb1) un rapport α:β de 5:1.

RMN $^1H$ (400 MHz, $CDCl_3$) pour les anomères α : δ 8,14-7,15 (m, 17Ha+17Hb, H-*aro*), 6,96 (d, 2Ha, *J* 9,0 Hz, H-*aroPMB*a), 6,71 (d, 2Hb, *J* 9,0 Hz, H-*aroPMB*b), 6,36-6,26 (m, 1Ha+1Hb, H-3a, H-3b), 6,02 (t, 1Ha, *J* 10,0 Hz, H-4a), 5,88 (d, 1Ha, *J* 3,5 Hz, H-1a), 5,86 (d, 1 Hb, *J* 3,5 Hz, H-1b), 5,85-5,74 (m, 1Ha+3Hb, H-5a, H-5b, C$H_2$PhO$CH_3$b), 5,63 (s, 2Ha, C$H_2$PhO$CH_3$a), 5,51-5,42 (m, 1Ha+1Hb, H-4b, H-2a), 5,31 (dd, 1Hb, *J* 3,5 Hz, *J* 10,0 Hz, H-2b), 3,82 (s, 3Hb, O$CH_3$b), 3,75 (s, 3Ha, O$CH_3$a).

RMN $^{13}C$ (100 MHz, $CDCl_3$) pour les anomères α : δ 165,8, 165,5, 164,7, 162,9, 150,5 (C=O, C=N), 133,7-124,8 *(C-aro),* 114,5 (C-*aroPMB*b), 114,2 (C-*aroPMB*a), 90,9 (C-1a), 90,8 (C-1b), 72,1 (C-2a), 71,8 (C-2b ou C-3b ou C-4b), 71,2 (C-4a), 70,2 (C-2b ou C-3b ou C-4b), 70,0 (C-3a), 69,3 (C-2b ou C-3b ou C-4b), 64,1 (C-5a), 63,4 (C-5b), 56,7 ($CH_2$PhO$CH_3$a), 55,4 (O$CH_3$b), 55,2 (O$CH_3$a), 52,0 Masse calculée pour $C_{35}H_{31}N_4O_9$ $[M+H]^+$ 651,2091, trouvée 651,2111.

**1.5. trichloroacétimidate de 2,3,4-tri-*O*-benzoyl-5-*C*-[2-(4-(méthoxybenzyl)-2*H*-tétrazol-5-yl]-α-D-xylopyranosyle (Vb2) et trichloroacétimidate de 2,3,4-tri-*O*-benzoyl-5-*C*-[1-(4-méthoxybenzyl)-1*H*-tétrazol-5-yl]-α-D-xylopyranosyle (Vb1)**

A une solution du mélange obtenu à l'étape 1.4 (6,0 g, 9,23 mmol) dans du dichlorométhane (170 mL) à température ambiante, sont ajoutés du 1,8-diazabicyclo[5.4.0] undec-7-ène (278 μL, 1,86 mmol) puis du trichloroacétonitrile (14,6 ml, 184 mmol). Le milieu réactionnel est agité 1 h à température ambiante. Une solution d'acide acétique (105 μL, 1,83 mmol) dans de l'eau (50 mL) est ajoutée. Les phases sont séparées, la phase organique est lavée avec de l'eau (50 mL) puis séchée sur sulfate de sodium. Le solvant est éliminé sous pression réduite et le résidu purifié par chromatographie sur gel de silice (silice préalablement neutralisée par des lavages avec une solution de triéthylamine, 5% dans de l'acétate d'éthyle (cyclohexane-acétate d'éthyle 7:3), pour donner un mélange d'isomères du produit attendu (4,7 g, 65%) sous forme de cristaux jaune. Le spectre de RMN du proton dans $CDCl_3$ montre un rapport (Vb2):(Vb1) de 3:1.

RMN $^1H$ (400 MHz, $CDCl_3$) : δ 8,77 (s, 1H-b, N*H*b), 8,69 (s, 1Ha, N*Ha*), 8,20-7,29 (m, 15Ha+17Hb, H-*aro*), 7,18 (d, 2Ha, *J* 8,5 Hz, H-*aroPMB*a), 6,99 (d, 2Hb, *J* 8,5 Hz, H-*aroPMBb*), 6,94 (m, 1Ha+1Hb, H-1 a, H-1 b), 6,74 (d, 2Ha, *J* 8,5 Hz, H-*aroPMB*a), 6,36 (t, 1Ha, *J* 10,0 Hz, H-3a), 6,29 (t, 1Hb, *J* 10,0 Hz, H-3b), 6,10 (t, 1Ha, *J* 10,0 Hz, H-4a), 5,80-5,69 (m, 2Ha+3Hb, H-2a, C$H_2$PhO$CH_3$b, H-5a, H-5b), 5,64 (s, 2Ha, C$H_2$PhO$CH_3$a), 5,62-5,55 (m, 2Hb, H-4b, H-2b), 3,84 (s, 3Hb, -O$CH_3$b), 3,75 (s, 3Ha, O$CH_3$a).

RMN $^{13}C$ (100 MHz, $CDCl_3$) : δ 165,6, 165,4, 164,4, 162,0, 160,5, 159,9 (C=O, C=N), 134,0-124,8 (C-*aro*), 114,6 (C-*aroPMB*b), 114,3 (C-*aroPMB*a), 93,1 (C-1a), 92,8 (C-1b), 70,9 (C-2b ou C-3b ou C-4b), 70,7 (C-4a), 70,5 (C-2a), 70,0 (C-2b ou C-3b ou C-4b), 69,8 (C-3a), 69,3 (C-2b ou C-3b ou C-4b), 66,9 (C-5a), 66,0 (C-5b), 56,5 ($CH_2$PhO$CH_3$a), 55,3 (O$CH_3$b), 55,1 (O$CH_3$a), 51,8 ($CH_2$PhO$CH_3$b).

**1.6. 3-*O*-pivaloyl-*N*-éthoxycarbonylnormorphin-6-yl 2,3,4-tri-*O*-benzoyl-5-*C*-[2-(4-méthoxybenzyl)-2*H*-tétrazol-5-yl]-β-D-xylopyranoside**

A une solution du mélange obtenu à l'étape 1.5 (3,6 g, 4,54 mmol) et de 3-*O*-pivaloyl-N-éthoxycarbonylnormorphine (1,0 g, 2,34 mmol) dans du dichlorométhane (50 mL) à 0°C sous argon, est ajouté du trifluorométhanesulfonyl de triméthylsilane (1,7 mL, 9,38 mmol). Le milieu réactionnel est agité 30 min à 0°C puis 30 min à température ambiante. De la N-diisopropyléthylamine (1 mL) est ajoutée, le mélange est agité 15 min puis concentré à sec sous pression réduite. Le résidu est purifié par chromatographie sur gel de silice (cyclohexane-acétate d'éthyle 3:2) pour donner le composé attendu (1,7 g, 69%) sous forme de cristaux blancs.

Point de fusion 185-188°C.

RMN $^1H$ (400 MHz, $CDCl_3$) : δ 7,97-7,28 (m, 15H, H-*aro*), 7,17 (m, 2H, H-*aroPMB*), 6,72 (m, 3H, H-*aroPMB,* H-1), 6,54 (d, 1H, *J* 8,5 Hz, H-2), 6,13 (t, 1H, *J* 10,0 Hz, H-4'), 5,90 (t, 1H, *J* 10,0 Hz, H-3'), 5,72 (m, 1 H, H-8), 5,67 (m, 1 H, H-2'), 5,61 (d, 2H, *J* 5,0 Hz, $CH_2$PhO$CH_3$), 5,43 (d, 1H, *J* 7,0 Hz, H-1'), 5,32 (d, 1H, *J* 10,0 Hz, H-5'), 5,25 (m, 1H, H-7), 5,00-4,80 (m, 2H, H-9, H-5), 4,40 (m, 1 H, H-6), 4,20 (m, 2H, O$CH_2$$CH_3$), 4,00 (m, 1H, H-16a), 3,74 (s, 3H, O$CH_3$), 3,00 (m, 1H, H-16b), 2,84-2,74 (m, 2H, H-10), 2,48 (m, 1H, H-14), 1,89 (m, 2H, H-15), 1,30 (m, 12H, C($CH_3$)$_3$, O$CH_2$$CH_3$).

RMN $^{13}C$ (100 MHz, $CDCl_3$) : δ 176,4, 165,7, 165,1, 164,5, 159,9 (C=O, C=N), 155,0, 150,5, 133,1, (C-*aro*),

130,7 (C-8), 129,8, 129,7, 129,6, 128,3, 128,2, 128,1, (C-*aro*), 127,4 (C-7), 122,2 (C-1), 119,3 (C-2), 114,3 (*C-aroPMB*), 99,4 (C-1'), 89,9 (C-5), 72,9 (C-3', C-6), 72,5 (C-2'), 71,5 (C-4'), 68,7 (C-5'), 61,6 ($OCH_2CH_3$), 56,6 ($CH_2PhOCH_3$), 55,2 ($OCH_3$), 50,2 (C-9), 44,3 (C-13), 39,8 (C-14), 37,2 (C-16), 35,3 (C-15), 35,0 ($C(CH_3)_3$), 30,2 (C-1 0), 29,8 ($C(CH_3)_3$), 14,7 ($OCH_2CH_3$).

Masse calculée pour $C_{59}H_{57}N_5O_{14}Na$ $[M+Na]^+$ 1082,3800, trouvée 1082,3802.

### 1.7. 3-*O*-pivaloyl-*N*-éthoxycarbonylnormorphin-6-yl 2,3,4-tri-*O*-benzoyl-5-*C*-(tétrazol-5-yl)-β-D-xylopyranoside

Une solution du composé obtenu à l'étape 1.6 (1,6 g, 1,51 mmol) dans de l'acide trifluoroacétique (4,5 mL, 60,6 mmol) est portée à reflux pendant 15 min. Le milieu réactionnel est concentré à sec, le résidu est repris dans du toluène (2 x 10 mL) et reconcentré. Le résidu est purifié par chromatographie sur gel de silice (cyclohexaneacétate d'éthyle 1:2) pour donner le composé attendu (850 mg, 60%) sous forme de cristaux de couleur jaune pâle.

Point de fusion 169-171°C.

RMN $^1H$ (400 MHz, $CDCl_3$) : δ 7,98-7,24 (m, 15H, H-*aro*), 6,76 (d, 1 H, *J* 8,0 Hz, H-1), 6,58 (d, 1H, *J* 8,0 Hz, H-2), 6,01 (t, 1H, *J* 10,0 Hz, H-3'), 5,98-5,95 (m, 1H, H-8), 5,72 (m, 1H, H-4'), 5,62 (m, 1H, H-2'), 5,41 (d, 1H, *J* 10,0 Hz, H-5'), 5,34-5,26 (m, 2H, H-1' et H-7), 5,02-4,96 (m, 1H, H-9), 4,85 (m, 1H, H-5), 4,38-4,31 (m, 1H, H-6), 4,22-4,13 (m, 2H, $OCH_2CH_3$). 4,07-3,98 (m, 1H, H-16a), 3,10-2,83 (m, 2H, H-16b, H-10a), 2,82-2,72 (m, 1H, H-10b), 2,47 (m, 1H, H-14), 1,93-1,85 (m, 2H, H-15), 1,47 (s, 9H, $C(CH_3)_3$), 1,33-1,25 (m, 3H, $OCH_2CH_3$).

RMN $^{13}C$ (100 MHz, $CDCl_3$) : δ 165,6, 165,1, 165,0 (C=O, C=N), 133,5, 132,4, 130,0, 129,8, 128,5, 128,4, 128,3 (C-*aro*, C-8, C-7), 122,5 (C-1), 119,9 (C-2), 90,9 (C-1'), 77,0 (C-6), 72,0 (C-2' ou C-3'), 71,9 (C-2' ou C-3'), 70,7 (C-4'), 68,3 (C-5'), 61,8 ($OCH_2CH_3$), 49,7 (C-9), 44,5 (C-13), 40,0 (C-14), 37,1 (C-16), 35,5 (C-15), 29,7 (C-10), 27,2 ($C(CH_3)_3$), 14,7 ($OCH_2CH_3$).

Masse calculée pour $C_{51}H_{49}N_5O_3Na$ $[M+Na]^+$ 962,3225, trouvée 962,3211.

### 1.8. Trifluoroacétate de morphin-6-yl 5-*C*-(tétrazol-5-yl)-β-D-xylopyranoside (composé n°2)

A une suspension d'hydrure de lithium et d'aluminium (300 mg, 7,91 mmol) dans de tétrahydrofurane (12 mL), est ajoutée une solution du composé obtenu à l'étape 1.7 (500 mg, 0,532 mmol) dans du tétrahydrofurane (12 mL). Le milieu réactionnel est agité pendant 1 h à reflux. De l'acétate d'éthyle est ajouté pour détruire l'excès d'hydrure de lithium et d'aluminium et le milieu est porté à pH 1 par ajout d'une solution d'acide chlorhydrique 1N. Le milieu réactionnel est concentré à sec. Le résidu est purifié une première fois sur colonne de chromatographie en phase inverse ($H_2O$ pure puis ($H_2O$+0,1%acide trifluoroacétique)-acétonitrile 80:20) pour éliminer les sels. Une deuxième purification par chromatographie préparative en phase inverse (gradient ($H_2O$+0,1%acide trifluoroacétique)- acétonitrile de 95:5 à 20:80) permet d'obtenir le composé attendu sous forme de cristaux blancs (42 mg, 16%).

Point de fusion 204-207°C.

RMN $^1H$ (400 MHz, $D_2O$) : δ 6,74 (d, 1H, *J* 8,0 Hz, H-1), 6,65 (d, 1H, *J* 8,0 Hz, H-2), 5,73 (m, 1H, H-8), 5,30 (m, 1H, H-7), 5,23 (d, 1H, *J* 5,5 Hz, H-5), 4,94-4,88 (m, 2H, H-1', H-5'), 4,48 (m, 1H, H-6), 4,15 (m, 1H, H-9), 3,80 (t, 1 H, *J* 9,5 Hz, H-4'), 3,69 (t, 1H, *J* 9,5 Hz, H-3'), 3,54 (m, 1H, H-2'), 3,37 (m, 1H, H-16a), 3,24 (m, 1H, H-10a), 3,10 (m, 1 H, H-16b), 2,95 (s, 3H, $NCH_3$), 2,95-2,83 (m, 2H, H-10b, H-14), 2,32-2,02 (m, 2H, H-15).

RMN $^{13}C$ (100 MHz, $D_2O$) : δ 145,5 (C=N), 137,8, 134,0 (C-*ipso*), 131,1 (C-8), 129,0 (C-ipso), 126,0 (C-7), 123,3 (C-ipso), 120,4 (C-2), 117,7 (C-1), 102,3 (C-1'), 88,1 (C-5), 75,0 (C-3'), 73,3 (C-6), 73,0 (C-2'), 72,4 (C-4'), 69,3 (C-5'), 60,5 (C-9), 47,1 (C-16), 41,5 (C-13), 40,9 ($NCH_3$), 38,4 (C-14), 32,4 (C-15), 20,8 (C-10).

Masse calculée pour $C_{23}H_{28}N_5O_7$ $[M+H]^+$ 486,1989, trouvée 486,1982.

## Exemple 2 : Trifluoroacétate de morphin-6-yl 5-*C*-(2-méthyl-1,3,4-oxadiazol-5-yl)-β-D-xylopyranoside (composé n°5)

### 2.1. 3-*O*-acétylmorphin-6-yl 2,3,4-tri-*O*-acétyl-5-*C*-(2-méthyl-1,3,4-oxadiazol-5-yl)-β-D-xylopyranoside

Une solution de trifluoroacétate de morphin-6-yl 5-*C*-(tétrazol-5-yl)-β-D-xylopyranoside (134 mg, 0,28 mmol), obtenu à l'étape 1.8 de l'exemple 1, dans de l'anhydride acétique (3 mL) est maintenue au reflux pendant une nuit. Le milieu réactionnel est concentré à sec, le résidu est repris dans du toluène (2 x 10 mL) et reconcentré. Le produit est purifié par chromatographie sur gel de silice (chloroformeméthanol de 98:2 à 95:5) pour donner le composé attendu (102 mg, 55%) sous forme de cristaux de couleur jaune pâle.

Point de fusion 180-186°C.

RMN $^1H$ (400 MHz, $CDCl_3$) : δ 6,77 (d, 1H, *J* 8,0 Hz, H-1), 6,59 (d, 1 H, *J* 8,0 Hz, H-2), 5,71 (m, 1H, H-8), 5,37 (m, 2H, H-4' et H-3'), 5,27 (m, 1H, H-7), 5,18 (m, 1H, H-2'), 4,99 (d, 1 H, *J* 7,5 Hz, H-1'), 4,95 (d, 1 H, *J* 5,5 Hz, H-5), 4,87 (m, 1 H, H-5'), 4,31 (m, 1 H, H-6), 3,71 (m, 1H, H-9), 3,07 (m, 1H, H-10a), 2,97-2,89 (m, 2H, H-16a, H-14), 2,62-2,52

(m, 8H, H-10b, $CH_3$oxadiazole, N$CH_3$, H-16b), 2,33 (s, $CH_3$CO), 2,28 (m, 1H, H-15a), 2,14 (s, 3H, $CH_3$CO), 2,04 (s, 3H, $CH_3$CO), 1,98 (m, 1H, H-15b), 1,93 (s, 3H, $CH_3$CO).

RMN $^{13}$C (100 MHz, $CDCl_3$) : δ 175,9, 170,0, 169,4, 169,3, 165,3, 161,2 (C=O, C=N), 150,3, 132,0 (C-*ipso*), 130,9 (C-8), 130,6, 130,4 (C-*ipso*), 127,5 (C-7), 122,6 (C-1), 119,5 (C-2), 100,0 (C-1'), 89,1 (C-5), 73,6 (C-6), 71,8 (C-3' ou C-4'), 71,0 (C-2'), 69,6 (C-3' ou C-4'), 67,9 (C-5'), 58,5 (C-9), 46,0 (C-16), 42,7 (C-13), 41,7 (N$CH_3$), 38,9 (C-14), 33,8 (C-15), 21,5 (C-10), 20,7, 20,6, 20,4 ($CH_3$CO), 11,0 ($CH_3$oxadiazole).

Masse calculée pour $C_{33}H_{38}N_3O_{12}$ $[M+H]^+$ 668,2455, trouvée 668,2560.

**2.2. Trifluoroacétate de morphin-6-yl 5-*C*-(2-méthyl-1,3,4-oxadiazol-5-yl)-β-D-xylopyranoside (composé n°5)**

A une solution de 3-*O*-acétylmorphin-6-yl 2,3,4-tri-*O*-acétyl-5-*C*-(2-méthyl-1,3,4-oxadiazol-5-yl)-β-D-xylopyranoside précédemment préparé (100 mg, 0,15 mmol) dans du méthanol (2 mL), à température ambiante, est ajouté du méthylate de sodium (32 mg, 0,59 mmol). Le milieu réactionnel est agité pendant 3 h à température ambiante puis neutralisé par addition de résine commercialisée sous la dénomination Amberlyte $H^+$® (Rohm and Haas). La solution est filtrée et le filtrat concentré sous pression réduite. Le résidu est purifié par chromatographie préparative en phase inverse (gradient ($H_2O$ + 0, 1 % acide trifluoroacétique) -acétonitrile de 95:5 à 20:80) pour donner le produit attendu (34 mg, 46%) sous forme de cristaux blancs.

Point de fusion 215-218°C.

RMN $^1$H (300 MHz, $D_2O$) : δ 6,83 (d, 1H, *J* 8,0 Hz, H-1), 6,74 (d, 1H, *J* 8,0 Hz, H-2), 5,84 (m, 1H, H-8), 5,46 (m, 1H, H-7), 5,32 (d, 1H, J 6,5 Hz, H-5), 5,00 (d, 1H, *J* 8,0 Hz, H-1'), 4,90 (m, 1H, H-5'), 4,59 (m, 1 H, H-6), 4,26 (m, 1H, H-9), 3,92 (t, 1 H, *J* 9,5 Hz, H-4'), 3,74 (t, 1H, *J* 9,5 Hz, H-3'), 3,59 (m, 1H, H-2'), 3,45-3,38 (m, 1H, H-16a), 3,29 (d, 1H, H-10a), 3,17 (m, 1H, H-16b), 3,03-2,92 (m, 5H, *N*$CH_3$, H-10b, H-14), 2,63 (s, 3H, $CH_3$oxadiazole), 2,40-2,11 (m, 2H, *H*-15).

RMN $^{13}$C (75 MHz, $D_2O$) : δ 131,2 (C-8), 126,3 (C-7), 120,6 (C-2), 118,2 (C-1), 102,5 (C-1'), 88,2 (C-5), 75,0 (C-3'), 73,5 (C-6), 73,0 (C-2' ou C-4'), 71,4 (C-2' ou C-4'), 69,3 (C-5'), 60,7 (C-9), 47,4 (C-6), 41,1 (N$CH_3$), 38,7 (C-14), 32,6 (C-15), 21,1 (C-10), 10.2 ($CH_3$oxadiazole).

Masse calculée pour $C_{25}H_{30}N_3O_8$ $[M+H]^+$ 500,2033, trouvée 500,2021.

**<u>Exemple 3</u> : Trifluoroacétate de morphin-6-yl 5-*C*-(5-(4-méthoxyphényl)-4-(2,2,2-trifluoroéthyl)-4*H*-1,2,4-triazol-3-yl]-β-D-xylopyranoside (composé n°6)**

**3.1. 1,2,3,4-tétra-*O*-benzoyl-5-*C*-[5-(4-méthoxyphényl)-4-(2,2,2-trifluoroéthyl)-4*H*-1,2,4-triazol-3-yl]-α/β-D-xylopyranose**

A une solution de 1,2,3,4-tétra-*O*-benzoyl-5-*C*-(tétrazol-5-yl)-α/β-D-xylopyranose (10,0 g, 15,77 mmol), obtenu à l'étape 1.2 de l'exemple 1, dans du toluène (119 mL) sont ajoutés de la triéthylamine (4,3 mL, 30,85 mmol) et du chlorure de 2,2,2-trifluoro-*N*-(4-méthoxybenzyl)acétimidoyle (7,9 g, 31,47 mmol). Le milieu réactionnel est agité une nuit à reflux. Le solvant est éliminé sous pression réduite et le résidu purifié par chromatographie sur gel de silice (cyclohexane-acétate d'éthyle 1:1) pour donner le produit attendu (7,3 g, 56%) sous forme de cristaux de couleur jaune pâle. Le spectre de RMN du proton dans $CDCl_3$ montre un rapport α:β de 5:2.

RMN $^1$H (400 MHz, $CDCl_3$) : δ 8,28-7,29 (m, 22Hα+22Hβ, H-*aro*), 7,00 (d, 2Hα, *J* 8,5 Hz, H-*aroPMP*α), 6,94 (d, 2Hβ, *J* 8,5 Hz, H-*aroPMP*β), 6,84 (d, 1Hα, *J* 3,5 Hz, H-1α), 6,54 (t, 1Hβ, *J* 8,5 Hz, H-4β), 6,45 (m, 2Hα, H-3α, H-4α), 6,38 (d, 1Hβ, *J* 7.0 Hz, H-1β), 6,18 (t, 1Hβ, *J* 8,5 Hz, H-3β), 5,94 (dd, 1Hβ, *J* 7,0 Hz, *J* 8,5 Hz, H-2β), 5,76-5,69 (m, 2Hα, H-2α, H-5α), 5,61 (d, 1Hβ, *J* 8,5 Hz, H-5β), 5,13 (m, 1Hβ, $CH_2CF_3$β), 4,99 (m, 1Hα, $CH_2CF_3$α), 4,65-4,51 (m, 1Hα+1Hβ, $CH_2CF_3$α, $CH_2CF_3$β), 3,84 (s, 3Hα, O$CH_3$α), 3,83 (s, 3Hβ, O$CH_3$β).

RMN $^{13}$C : (100 MHz, $CDCl_3$) δ 166,0, 165,4, 165,2, 164,7 (C=O), 161,5, 156,8, 149,5 (C=N, C-*ipsoPMP*), 134,4-128,2 (C-*aro*), 117,5 (C-*ipsoPMP*), 114,6 (C-*aroPMP*α), 114,5 (C-*aroPMP*β), 93,2 (C-1β), 90,1 (C-1α), 71,9 (C-2β ou C-3β ou C-4β), 70,3 (C-2α ou C-2α ou C-4α), 70,2 (C-2α ou C-2α ou C-4α), 70,0 (C-2β ou C-3β ou C-4β), 69,2 (C-2a ou C-2α ou C-4α), 69,1 (C-2β ou C-3β ou C-4β), 66,1 (C-5α, C-5β), 55,4 (O$CH_3$α, O$CH_3$β), 45,4 ($CH_2CF_3$β), 45,0 ($CH_2CF_3$α).

Masse calculée pour $C_{44}H_{35}F_3N_3O_{10}$ $[M+H]^+$ 822,2275, trouvée 822,2291.

**3.2. 2,3,4-tri-*O*-benzoyl-5-*C*-[5-(4-méthoxyphényl)-4-(2,2,2-trifluoroéthyl)-4*H*-1,2,4-triazol-3-yl]-α/β-D-xylopyranose**

A une solution du composé préparé à l'étape 3.1 (7,2 g, 8,77 mmol) dans du *N,N*-diméthylformamide (50 mL), à 0°C, est ajouté de l'acétate d'hydrazine (1,2 g, 13,0 mmol) par petites portions en 15 min. Le milieu réactionnel est agité 1 h à 0°C puis 2,5 h à température ambiante. De l'acétate d'éthyle (100 mL) et de l'eau (50 mL) sont ajoutés. Les

phases sont séparées. La phase aqueuse est réextraite avec de l'acétate d'éthyle (1 x 50 mL). Les phases organiques réunies sont séchées sur sulfate de sodium. Le solvant est éliminé sous pression réduite et le résidu purifié par chromatographie sur gel de silice (cyclohexane-acétate d'éthyle 2:3) pour donner le produit attendu (3,1 g, 49%) sous forme de cristaux blancs. Le spectre de RMN du proton dans $CDCl_3$ montre un rapport α:β de 9:1.

RMN $^1H$ (400 MHz, $CDCl_3$) pour l'anomère α δ 7,99-7,26 (m, 17H, H-*aro*), 6,76 (d, 2H, *J* 9,0 Hz, H-*aroPMP*), 6,24 (t, 1H, *J* 10,0 Hz, H-3), 6,14 (t, 1H, *J* 10,0 Hz, H-4), 5,80 (d, 1 H, *J* 10,0 Hz, H-5), 5,45 (d, 1H, *J* 3,0 Hz, H-1), 5,31 (m, 1 H, $CH_2CF_3$), 5,07 (dd, 1 H, *J* 3,0 Hz, *J* 10,0 Hz, H-2), 4,92 (m, 1H, $CH_2CF_3$), 3,72 (s, 3H, $OCH_3$).

RMN $^{13}C$ (100 MHz, $CDCl_3$) pour l'anomère α : δ 165,8, 165,5, 165,1 (C=O) 161,3, 156,5, 151,3 (C=N, C-*ipsoPMP*), 133,2-128,2 (C-aro), 122,9 (C-*ipso*) 117,6 (C-*ipsoPMP*), 114,4 (C-*aroPMP*), 90,2 (C-1), 72,1 (C-2), 70,8 (C-3), 69,6 (C-4), 62,1 (C-5), 55,2 ($OCH_3$), 45,3 (m, $CH_2CF_3$).

Masse calculée pour $C_{37}H_{31}F_3N_3O_9$ $[M+H]^+$ 718,2012, trouvée 718,2034.

### 3.3. Trichloroacétimidate de 2,3,4-tri-*O*-benzoyl-5-*C*-[5-(4-méthoxyphényl)-4-(2,2,2-trifluoroéthyl)-4*H*-1,2,4-triazol-3-yl]-α/β-D-xylopyranosyle

A une solution du produit préparé à l'étape 3.2 (2,8 g, 3,91 mmol) dans du dichlorométhane (65 mL) à température ambiante, sont ajoutés du 1,8-diazabicyclo[5.4.0]undec-7-ène (115 μL, 0,770 mmol) puis du trichloroacétonitrile (7,8 ml, 77,8 mmol). Le milieu réactionnel est agité 1 h à température ambiante. Une solution d'acide acétique (45 μL, 0,786 mmol) dans de l'eau (25 mL) est ajoutée. Les phases sont séparées, la phase organique est lavée par de l'eau (25 mL) puis séchée sur sulfate de sodium. Le solvant est éliminé sous pression réduite et le résidu purifié par chromatographie sur gel de silice (silice préalablement neutralisée par des lavages avec une solution de triéthylamine, 5% dans de l'acétate d'éthyle) (cyclohexane-acétate d'éthyle 7:3) pour donner le produit attendu (2,1 g, 63 %) sous forme de cristaux blancs. Le spectre RMN du proton dans $CDCl_3$ montre un rapport α:β de 5:4.

RMN $^1H$ (400 MHz, $CDCl_3$) : δ 8,78 (s, 1Hβ, N*H*β), 8,77 (s, 1Hα, N*H*α), 8,00-7,31 (m, 17Hα+17Hβ, H-*aro*), 7,00 (m, 2Hα+2Hβ, H-*aroPMP*α, H-*aroPMP*β), 6,78 (d, 1Hα, *J* 3,5 Hz, H-1α), 6,51 (t, 1Hα, *J* 10,0 Hz, H-4α), 6,44-6,34 (m, 1Hα+1Hβ, H-3α, H-4β), 6,21 (d, 1Hβ, *J* 8,0 Hz, H-1β), 6,13 (t, 1Hβ, *J* 9,5 Hz, H-3β), 5,92 (dd, 1Hβ, *J* 8,0 Hz, *J* 9,5 Hz, H-2β), 5,69 (d, 1Hα, *J* 10,0 Hz, H-5α), 5,62 (dd, 1Hα, *J* 3,5 Hz, *J* 10,0 Hz, H-2α), 5,52 (d, 1Hβ, *J* 9,5 Hz, H-5β), 5,27 (m, 1Hβ, $CH_2CF_3$β), 5,00 (m, 1Hα, $CH_2CF_3$α), 4,58 (m, 1Hα+1Hβ, $CH_2CF_3$α, $CH_2CF_3$β), 3,87 (s, 3Hα+3Hβ, $OCH_3$α, $OCH_3$β).

RMN $^{13}C$ (100 MHz, $CDCl_3$) δ 165,7, 165,4, 164.7, 164,4, (C=O) 161.4, 149.2 (C=N, C-*ipsoPMP*), 133,7-128,2 (C-aro), 117,7 (C-*ipsoPMP*), 114.5 (C-*aroPMP*), 96,6 (C-1β), 93,5 (C-1α), 72,5, 70,5, 70.2, 70,0, 69,3, 68,9 (C-2α, C-3β, C-3α, C-3β, C-4α, C-4β), 66,2 (C-5α, C-5β), 55,4 ($OCH_3$α, $OCH_3$β), 45,1 (m, $CH_2CF_3$α, $CH_2CF_3$β).

### 3.4. 3-*O*-pivaloyl-*N*-éthoxycarbonylnormorphin-6-yl 2,3,4-tri-*O*-benzoyl-5-*C*-[5-(4-méthoxyphényl)-4-(2,2,2-trifluoroéthyl)-4*H*-1,2,4-triazol-3-yl]-β-D-xylopyranoside

A une solution du produit préparé à l'étape 3.3 (1,20 g, 1,40 mmol) et de 3-*O*-pivaloyl-*N*-éthoxycarbonylnormorphine (410 mg, 0,96 mmol) dans du dichlorométhane (50 mL) à 0°C sous argon, est ajouté du trifluorométhanesulfonyl de triméthylsilane (696 μL, 3,84 mmol). Le milieu réactionnel est agité 30 min à 0°C puis 30 min à température ambiante. De la N-diisopropyléthylamine (0,5 mL) est ajoutée, le mélange est agité 15 min puis concentré à sec sous pression réduite. Le résidu est purifié par chromatographie sur gel de silice (cyclohexane-acétate d'éthyle 3:7) pour donner le composé attendu (680 mg, 63%) sous forme de cristaux orangés.

Point de fusion 194°C.

RMN $^1H$ (400 MHz, $CDCl_3$) δ 7,98-7,29 (m, 17H, H-*aro*), 7,03 (d, 2H, J 9,0 Hz, H-*aroPMP),* 6,71 (d, 1 H, *J* 8,0 Hz, H-1), 6,54 (d, 1 H, J 8,0 Hz, H-2), 6,00 (m, 2H, H-3', H-4'), 5,73 (m, 1H, H-8), 5.66 (m, 1H, H-2'), 5,41 (m, 2H, H-1', H-5'), 5,30 (m, 1H, H-7), 4,98 (m, 2H, H-9, $CH_2CF_3$), 4,86-4,76 (m, 2H, H-5, $CH_2CF_3$), 4,30 (m, 1H, H-6), 4,17 (m, 2H, $OCH_2CH_3$), 4,01 (m, 1H, H-16a), 3,89 (s, 3H, $OCH_3$), 3,07-2,84 (m, 2H, H-16b, H-10a), 2,75 (m, 1H, H-10b), 2,47 (m, 1H, H-14), 1,88 (m, 2H, H-15), 1,27 (m, 12H, $C(CH_3)_3$, $OCH_2CH_3$).

RMN $^{13}C$ (100 MHz, $CDCl_3$) δ 165,6, 165,1, 165,0, (C=O) 161,4, 156,9, 149,6 (C=N, C-*ipsoPMP*), 133,4 (C-*aro*), 130,7 (C-8), 129,9-127,8 (C-*aro*, C-7), 122,5 (C-1), 118,0 (C-*ipsoPMP*), 119,4 (C-2), 114,6 (C-*aroPMP*), 100,6 (C-1'), 90,2 (C-5), 74,0 (C-6), 72,3 (C-2' ou C-3' ou C-4'), 71,8 (C-2' ou C-3' ou C-4'), 70,3 (C-2', C-3' ou C-4'), 69,9 (C-5'), 61,7 ($OCH_2CH_3$), 55,4 ($OCH_3$), 48,1 (C-9), 44,5 ($CH_2CF_3$ ou C-13), 39,8 (C-14), 37,2 (C-16), 35,3 (C-15), 35,0 ($C(CH_3)_3$), 30,0 (C-10), 27,1 ($C(CH_3)_3$), 14,7 ($OCH_2CH_3$).

Masse calculée pour $C_{61}H_{58}F_3N_4O_{14}$ $[M+H]^+$ 1127,3902, trouvée 1127,3889.

**3.5. Trifluoroacétate de morphin-6-yl 5-*C*-[5-(4-méthoxyphényl)-4-(2,2,2-trifluoroéthyl)-4*H*-1,2,4-triazol-3-yl]-β-D-xylopyranoside (composé n°6)**

A une suspension d'hydrure de lithium et d'aluminium (311 mg, 8,19 mmol) dans du tétrahydrofurane (13 mL), est ajoutée une solution du composé obtenu à l'étape 3.4 (622 mg, 0,55 mmol) dans du tétrahydrofurane (13 mL). Le milieu réactionnel est agité pendant 1 h à reflux. De l'acétate d'éthyle est ajouté pour détruire l'excès de $LiAlH_4$ et le milieu est porté à pH 1 par ajout d'une solution d'acide chlorhydrique 1 N. Le milieu réactionnel est concentré à sec. Le résidu est purifié par deux passages successifs sur colonne de chromatographie en phase inverse ($H_2O$ pure puis ($H_2O$+0,1%acide trifluoroacétique)-$CH_3CN$ 80:20) pour éliminer les sels. Une dernière purification par chromatographie préparative en phase inverse (gradient ($H_2O$+0,1% acide trifluoroacétique)-acétonitrile de 95:5 à 20:80) permet d'obtenir le composé attendu sous forme de cristaux blancs (35 mg, 10%).

Point de fusion 274-276°C.

RMN $^1$H (400 MHz, $D_2O$) : δ 7,51 (d, 2H, *J* 8,5 Hz, H-*aroPMP*), 7,15 (d, 2H, J 8,5 Hz, H-*aroPMP*), 6,72 (d, 1H, *J* 8,0 Hz, H-1), 6,64 (d, 1H, *J* 8,0 Hz, H-2), 5,67 (m, 1H, H-8), 5,29 (m, 1 H, H-7), 5,22 (d, 1 H, *J* 6,0 Hz, H-5), 5,09-4,90 (m, 3H, $CH_2CF_3$, H-1'), 4,82 (d, 1H, J 9,5 Hz, H-5'), 4,41 (m, 1 H, H-6), 4,18 (m, 1H, H-9), 4,12 (t, 1H, J 9,5 Hz, H-4'), 3,88 (s, 3H, O$CH_3$), 3,71 (t, 1H, *J* 9,5 Hz, H-3'), 3,57 (dd, 1H, *J* 8,0 Hz, *J* 9,5 Hz, H-2'), 3,36 (m, 1H, H-16a) 3,26 (m, 1H, H-10a), 3,12-3,03 (m, 1H, H-16b), 2,96 (s, 3H, N-$CH_3$), 2,93-2,84 (m, 2H, H-10b, H-14), 2,31-2,22 (m, 2H, H-15).

RMN $^{13}$C (100 MHz, $D_2O$) : δ 130,9 (C-*aroPMP*), 126,0 (C-7), 120,4 (C-2), 117,7 (C-1), 116,8 (C-*ipsoPMP*), 114,7 (C-*aroPMP*), 102,6 (C-1'), 88,1 (C-5), 75,0 (C-3'), 73,6 (C-6), 72,8 (C-2'), 72,0 (C-4'), 67,9 (C-5'), 60,5 (C-9), 55,4 (O$CH_3$), 47,2 (C-16), 46,6 ($CH_2CF_3$), 40,9 (N$CH_3$), 38,4 (C-14), 32,4 (C-15), 20,8 (C-10).

Masse calculée pour $C_{33}H_{26}F_3N_4O_8$ $[M+H]^+$ 673,2485, trouvée 673,2482.

<u>Exemple 4:</u> **Trifluoroacétate de morphin-6-yl 5-C-(tétrazol-5-yl)-α/β-D-xylopyranoside (composé n°1)**

**4.1 3-*O*-pivaloyl-*N*-éthoxycarbonylnormorphin-6-yl 2,3,4-tri-*O*-benzoyl-5-*C*-(tétrazol-5-yl)-aJp-D-xylopyranoside**

Une solution de 3-*O*-pivaloyl-*N*-éthoxycarbonylnormorphin-6-yl 2,3,4-tri-*O*-benzoyl-5-*C*-[2-(4-méthoxybenzyl)-2*H*-tétrazol-5-yl]-β-D-xylopyranoside (1,6 g, 1,51 mmol), obtenu à l'étape 1.6 de l'exemple 1, dans de l'acide trifluoroacétique (4,5 mL, 60,6 mmol) est portée à reflux pendant 45 min. Le milieu réactionnel est concentré à sec, le résidu est repris dans du toluène (2 x 10 mL) et de nouveau concentré. Une filtration sur gel de silice (acétate d'éthyle) permet d'éliminer les impuretés apolaires. Le résidu (1,3 g) est engagé dans l'étape de réduction sans purification supplémentaire.

**4.2 trifluoroacétate de morphin-6-yl 5-*C*-(tétrazol-5-yl)-α/β-D-xylopyranoside (composé n°1)**

Le trifluoroacétate de morphin-6-yl 5-C-(tétrazol-5-yl)-α/β-D-xylopyranoside est synthétisé à partir du 3-*O*-pivaloyl-*N*-éthoxycarbonylnormorphin-6-yl 2,3,4-tri-*O*-benzoyl-5-*C*-(tétrazol-5-yl)-α/β-D-xylopyranoside obtenu précédemment (1,30 g, 1,38 mmol) selon la même procédure que celle décrite à l'étape 1.8 de l'exemple 1.
On obtient le mélange d'anomères attendu sous forme de cristaux jaune pâle (215 mg, 32%). Le spectre de RMN du proton dans $D_2O$ montre un rapport α:β de 1:1.

RMN $^1$H (400 MHz, $D_2O$) : δ 6,83-6,79 (m, 1Hα+1Hβ, H-1α, H-1β), 6,70 (m, 1Hα+1Hβ, H-2α, H-2β), 5,91 (m, 1Hα, H-8α), 5,82 (m, 1Hβ, H-8β), 5,60 (d, 1Hα, *J* 10.0Hz, H-5'α), 5,48 (m, 1Hα, H-7α), 5,39 (m, 1Hβ, H-7β), 5,31 (d, 1Hα, *J* 4,0 Hz, H-1'α), 5,28 (d, 1Hβ, *J*6,0 Hz, H-5β), 5,13 (d, 1Hα, *J* 6.0 Hz, H-5α), 5,01-4,96 (m, 2Hβ, H-5'β, H-1'β), 4,55 (m, 1Hβ, H-6β), 4,43 (m, 1Hα, H-6α), 4,26-4,17 (m, 1Hα+1Hβ, H-9α, H-9β), 3,99 (t, 1Hα, *J* 9,5 Hz, H-3'α), 3,82 (dd, 1Hα, *J* 4,0 Hz, *J* 9,5 Hz, H-2'α), 3,78-3,74 (m, 1Hα+2Hβ, H-4'α, H-3'β, H-4'β), 3,59 (m, 1Hβ, H-2'β), 3,41-3,38 (m, 1Hα+1Hβ, H-16aα, H-16aβ), 3,33-3,27 (m, 1Hα+1Hβ, H-10aα, H-10aβ), 3,17-3,08 (m, 1Hα+1Hβ, H-16bα, H-16bβ), 3,00 (s, 3Hα+3Hβ, *N$CH_3$*α, *N$CH_3$*β), 3,00-2,88 (m, 2Hα+2Hβ, H-10bα, H-10bβ, H-14a, H-14β), 2,37-2,07 (m, 2Hα+2Hβ, H-15α, H-15β).

RMN $^{13}$C (100 MHz, $D_2O$) : δ 136,5 (C-*ipso*), 131,2 (C-8α), 130,4 (C-8β), 129,0 (C-*ipso*), 126,5 (C-7α), 126,1 (C-7β), 123,3 (C-*ipso*), 120,5 (C-2α, C-2β), 117,8 (C-1α, C-1β). 102,5 (C-1'β), 100,0 (C-1'α), 90,1 (C-5α), 88,2 (C-5β), 74,9, 74,7, 73,6, 72,9, 72,5, 72,4, 72,3, 71,1 (C-2'α, C-2'β, C-3'α, C-3'β, C-4'α, C-4'β, C-6α, C-6β), 68,9 (C-5'β), 65,6 (C-5'α), 60,5 (C-9α, C-9β), 47,2 (C-16α, C-16β), 41,5 (C-13α, C-13β), 40,9 (N$CH_3$α, N$CH_3$β), 38,7 (C-14α), 38,4 (C-14β), 32,4 (C-15α, C-15β), 20,9 (C-10α, C-10β).

Masse calculée pour $C_{23}H_{28}N_5O_7$ $[M+H]^+$ 486,1989, trouvée 486,1979.

**Exemple 5 : Trifluoroacétate de morphin-6-yl 5-*C*-(2-méthyl-1,3,4-oxadiazol-5-yl)-α-D-xylopyranoside (composé n°4)**

**5.1 Trifluoroacétate de 3-*O*-acétyl-morphin-6-yl 2,3,4-tri-*O*-acétyl-5-C-(2-méthyl-1,3,4-oxadiazol-5-yl)-α-D-xylopyranoside et trifluoroacétate de 3-*O*-acétyl-morphin-6-yl 2,3,4-tri-*O*-acétyl-5-*C*-(2-méthyl-1,3,4-oxadiazol-5-yl)-α/β-D-xylopyranoside**

Une solution de trifluoroacétate de morphin-6-yl 5-C-(tétrazol-5-yl)-α/β-D-xylopyranoside (300 mg, 0,62 mmol), obtenu à l'étape 4.2 de l'exemple 4, dans de l'anhydride acétique (7 mL) est maintenue au reflux pendant une nuit. Le milieu réactionnel est concentré à sec, le résidu est repris dans du toluène (2 x 10 mL) et de nouveau concentré. Le produit est purifié sur colonne de silice ($CHCl_3$-$CH_3OH$ de 98:2 à 95:5) pour donner deux fractions :

- l'une contenant l'anomère a pur (45 mg)
- l'autre contenant un mélange des anomères α et β (92 mg). Le spectre de RMN du proton dans $CDCl_3$ montre un rapport α:β de 2:3 estimé par RMN et par HPLC.

Le rendement global de la réaction est de 33%.
RMN $^1H$ (400 MHz, $CDCl_3$) pour l'anomère α : δ 6,82 (d, 1H, *J* 8,0 Hz, H-1), 6,61 (d, 1 H, *J* 8,0 Hz, H-2), 5,74 (m, 1 H, H-8), 5,69 (t, 1 H, *J* 10,0 Hz, H-3'), 5,63 (d, 1 H, *J* 10,0 Hz, H-5'), 5,40 (d, 1 H, J 4,0 Hz, H-1'), 5,37-5,30 (m, 2H, H-4', H-7), 5,01-4,96 (m, 2H, H-2', H-5), 4,14 (m, 1H, H-6), 3,60 (m, 1 H, H-9), 3,10 (m, 1H, H-10a), 2,80 (m, 1H, H-16a), 2,70-2,50 (m, 9H, *$CH_3$oxadiazole,* N*$CH_3$*, H-10b, H-14, H-16b), 2,34 (s, 3H, *$CH_3$*CO), 2,31-2,25 (m, 1H, H-15a), 2,08 (s, 3H, *$CH_3$*CO), 2,05 (s, 3H, *$CH_3$*CO), 2,00 (m, 1H, H-15b), 1,95 (s, 3H, *$CH_3$*CO).
RMN $^{13}C$ (100 MHz, $CDCl_3$) pour l'anomère α : δ 170,2,169,2,168,6, 164,9, 162,2 (C=O, C=N), 122,7 (C-1), 119,4 (C-2), 97,6 (C-1'), 91,4 (C-5), 76,7 (C-6), 70,6 (C-2'), 70,0 (C-4'), 69,2 (C-3'), 64,2 (C-5'), 59,2 (C-9), 46,4 (C-16), 42,5 (N*$CH_3$*), 33,5 (C-15), 21,3 (C-10), 20,7, 20,6, 20,5 (*$CH_3$*CO), 11,0 (*$CH_3$-oxadiazole*).
Masse calculée pour $C_{33}H_{38}N_3O_{12}$ $[M+H]^+$ 668,2455, trouvée 668,2560.

**5.2 Trifluoroacétate de morphin-6-yl 5-*C*-(2-méthyl-1,3,4-oxadiazol-5-yl)-α-D-xylopyranoside (composé n°4)**

A une solution de trifluoroacétate de 3-*O*-acétyl-morphin-6-yl 2,3,4-tri-*O*-acétyl-5-C-(2-méthyl-1,3,4-oxadiazol-5-yl)-α-D-xylopyranoside (45 mg, 0,07mmol) dans du méthanol (0,5 mL), à température ambiante, est ajouté du méthylate de sodium (14 mg, 0,26 mmol). Le milieu réactionnel est agité pendant 3 heures à température ambiante puis neutralisé par addition de résine Amberlyte $H^+$® (Rohm and Haas). La solution est filtrée et le filtrat concentré sous pression réduite pour donner le trifluoroacétate de morphin-6-yl 5-C-(2-méthyl-1,3,4-oxadiazol-5-yl)-α-D-xylopyranoside (31 mg, 92%) sous forme de cristaux de couleur jaune pâle.
RMN $^1H$ (400 MHz, $CD_3OD$) pour l'anomère α:δ 8,54 (s, 1 H, Ph*OH*), 6,57 (d, 1 H, J 8,0 Hz, H-1), 6,46 (d, 1 H, J 8,0 Hz, H-2), 5,86 (m, 1H, H-8), 5,49 (d, 1H, J 10,0 Hz, H-5'), 5,39 (m, 1H, H-7), 5,13 (d, 1H, *J* 4,0 Hz, H-1'), 4,95 (d, 1 H, J 6,0 Hz, H-5), 4,24 (m, 1 H, H-6), 3,84 (t, 1 H, *J* 9,5 Hz, H-3'), 3,75 (dd, 1 H, *J* 9,5 Hz, *J* 10,0 Hz, H-4'), 3,60-3,55 (m, 2H, H-9, H-2'), 3,08 (m, 1H, H-10a), 2,76-2,70 (m, 2H, H-14, H-16a), 2,59 (s, 3H, *N$CH_3$*), 2,56 (s, 3H, *$CH_3$oxadiazole),* 2,55-2,45 (m, 2H, H-10b, H-16b), 2,12 (m, 1H, H-15a), 1,84 (m, 1H, H-15b).
RMN $^{13}C$ (100 MHz, $CD_3OD$) pour l'anomère α:δ 166,4, 166,3 (C=N), 147,6, 140,0 (C-*ipso*), 131,6 (C-8), 130,9 (C-*ipso*), 129,1 (C-7), 125,5 (C-*ipso*), 120,6 (C-2), 118,2 (C-1), 102,1 (C-1'), 92,1 (C-5), 78,0 (C-6), 74,7 (C-3'), 73,4 (C-2'), 73,3 (C-4'), 67,6 (C-5'), 60,9 (C-9), 47,7 (C-13), 44,6 (C-16), 42,5 (N*$CH_3$*), 40,7 (C-14), 35,4 (C-10), 22,3 (C-15), 10,8 (*$CH_3$oxadiazole*).
Masse calculée pour $C_{25}H_{30}N_3O_8$ $[M+H]^+$ 500,2033, trouvée : 500,2029 (α).

**Exemple 6 : Trifluoroacétate de morphin-6-yl 5-*C*-(2-méthyl-1,3,4-oxadiazol-5-yl)-α/β-D-xylopyranoside (composé n°3)**

Le même protocole que celui décrit à l'étape 5.2 de l'exemple 5 est utilisé à partir du mélange 3-*O*-acétyl-morphin-6-yl 2,3,4-tri-*O*-acétyl-5-*C*-(2-méthyl-1,3,4-oxadiazol-5-yl)-α/β-D-xylopyranoside (α:β 2:3) (92 mg, 0,14 mmol) obtenu à l'étape 5.1 de l'exemple 5. Le mélange d'anomères attendu est obtenu sous forme de cristaux de couleur jaune pâle (62 mg, 90%). Le spectre de RMN du proton dans $CDCl_3$ montre un rapport α:β de 2:3 estimé par RMN et par HPLC.
Masse calculée pour $C_{25}H_{30}N_3O_8$ $[M+H]^+$ 500,2033, trouvée : 500,2585 (α:β 2:3).
Le tableau qui suit illustre les structures chimiques et les propriétés physiques de quelques exemples de composés selon l'invention. Dans ce tableau:

- dans la colonne "anomère", "α" et "β" représentent respectivement les anomères α et β purs et "α/β" représente un

mélange de ceux-ci, le rapport entre parenthèses étant le rapport ($\alpha$:$\beta$);

- dans la colonne "masse", figure le résultat de masse du composé obtenu selon la méthode d'ionisation chimique;

- dans la colonne "point de fusion" est indiquée la valeur du point de fusion du composé en degré Celsius;
- dans la colonne "$\alpha_D$", est indiqué le pouvoir rotatoire du composé en degré; sont indiqués entre parenthèses le solvant et la concentration de la mesure; et
- dans la colonne "sel", "$CF_3COO^-$" représente un composé sous forme de trifluoroacétate.

**Tableau 1**

| N° | R1 | Anomère | Masse | PF(°C) Point de fusion | $\alpha_D$ | Sel |
|---|---|---|---|---|---|---|
| 1 | tétrazole | $\alpha/\beta$ (1/1) | 486,1979 | - | - | $CF_3COOH$ |
| 2 | tétrazole | $\beta$ | 486,1982 | 204 -207 | -116 (c=0,5, $CH_3OH$) | $CF_3COOH$ |
| 3 | 2-méthl-1,3,4-oxadiazole | $\alpha/\beta$ (2/3) | 500,2585 | - | - | $CF_3COOH$ |
| 4 | 2-méthyl-1,3,4-oxadiazole | $\alpha$ | 500,2029 | - | - | $CF_3COOH$ |
| 5 | 2-méthyle-1,3,4-oxadiazole | $\beta$ | 500,2021 | 215-218 | - | $CF_3COOH$ |
| 6 | 5(4-méthoxyphényl) - 4-(2,2,2-trifluoroéthyle) 1,2,4 triazole | $\beta$ | 673,2482 | 274-276 | - 72 (c=0.5, $CH_3OH$) | $CF_3COOH$ |

**Activité biologique**

Les composés selon l'invention ont fait l'objet d'essais pharmacologiques permettant de déterminer leur effet analgésique.

Des essais consistant à mesurer l'activité *in vivo* des composés de l'invention sur une réponse réflexe nociceptive ont été effectués. Dans cette approche, la latence de la réponse réflexe nociceptive de l'animal est mesurée comme témoin de la douleur.

**Test du « Tail-Flick »**

Mode opératoire

L'activité analgésique a été déterminée par le test du « Tail- Flick » chez la souris male Swiss (Iffa Credo). Ce test est basé sur le réflexe nociceptif spontané de retrait de la queue de l'animal provoqué par un stimulus thermique douloureux (source infra-rouge). Le test du « Tail-flick » (test de D'Amour et Smith, 1941, Pharmacol Exp Ther;72 : 74-79) consiste, après administration d'un produit, à placer la queue d'une souris au point focal de la source infrarouge de façon à produire un stimulus thermique nociceptif (température de surface d'environ 55-60°C). Le temps de réaction (TR) de la souris (latence entre le moment où le faisceau lumineux est déclenché et le moment où la souris retire sa queue) a été mesuré en duplicate à différents temps allant de 20 minutes à 120 minutes après l'administration du produit. L'intensité de chaleur est réglée de façon à ce que ce réflexe de retrait soit compris entre 0,5 et 3,5 secondes chez des animaux témoins et représente arbitrairement le critère pour une analgésie minimale (0 %). Deux mesures de temps de réaction ont été réalisées avant administration du produit pour chaque souris et permettent d'établir un temps de mesure de base. Un temps maximum de 8 secondes a été choisi comme temps maximum de réaction de manière à ne pas induire de dommage tissulaire par brulure chez les animaux et représente arbitrairement le critère pour une analgésie maximale (100 %). Le temps de réaction est augmenté par les analgésiques par rapport à un animal témoin ne recevant pas de traitement. Les produits ont été administrés par voie sous-cutanée et orale à des doses comprises entre 1,25 et 30 mg/kg (exprimées en sel).

Résultats

Les résultats obtenus pour les composés de l'invention sont présentés dans le tableau 2 dans lequel figurent les données suivantes :

- le pourcentage d'activité analgésique maximale (index % MPE max) obtenue pour chaque composé (à une dose testée),
- la $DE_{50}$ (exprimée en mg/kg) correspondant à la dose efficace pour chaque composé pour laquelle une analgésie de 50 % à été obtenue ; celle-ci est calculée à un temps donné après l'administration des composés ; et
- la durée de l'action analgésique à une dose donnée.

Le pourcentage d'activité analgésique (% MPE) est déterminé par la formule suivante :

**% MPE = (TRpost-administration - TRpré-administration)*100/(TRmax - TR pré-administration)**

**Tableau 2**

| N° | Composé (R1 et type d'anomère concerné) | %MPE max (dose, mg/kg, sc) | $DE_{50}$ 60 min. post-administration, mg/kg | Durée d'action, minutes (dose, mg/kg. sc) |
|---|---|---|---|---|
| 1 | (tétrazole $\alpha/\beta$) | 50 (10) | 10 | >120 (10) |
| 2 | (tétrazole $\beta$) | 100 (1,25) | <1,25 | >120 (1,25) |
| 3 | (2-méthyl-1,3,4-oxadiazole $\alpha/\beta$) | 100 (5) | 3,2 | >120 (5) |
| 4 | (2-méthyl-1,3,4-oxadiazole $\alpha$) | 65 (10) | 8 | 90 (10) |
| 5 | (2-méthyl-1,3,4-oxadiazole $\beta$) | 100 (1,25) | <1,25 | >120 (1,25) |
| 6 | (5(4-méthoxyphényl)-4_(2,2,2-trifluoroéthyle) 1,2,4 triazole $\beta$) | - | - | - |

Les résultats indiquent que les composés selon l'invention ont des activités analgésiques puissantes (>50 %) pour des doses comprises entre 1,25 mg/kg et 10 mg/kg si ils sont administrés par voie sous-cutanée (mesurée 60 minutes post-administration) et pour des doses comprises entre au moins 2,5 et 30 mg/kg si ils sont administrés par voie orale (mesurée 60 minutes post-administration).

Ces activités analgésiques sont persistantes et de longues durées, supérieures à 120 minutes après leur administration à dose unique.

Parmi les composés les plus actifs dont l'activité a été évaluée par voie orale, à titre d'exemple le composé n°5 présente un pourcentage d'activité analgésique maximale (index % MPE max) de 78 pour la dose de 10 mg/kg et une DE50 à 60 minutes inférieure à 2,5 mg/kg.

Il apparaît donc que les composés selon l'invention ont une activité analgésique.

Les composés selon l'invention peuvent donc être utilisés pour la préparation de médicaments, en particulier de médicaments destinés au traitement ou à la prévention de la douleur.

Ainsi, selon un autre de ses aspects, l'invention a pour objet des médicaments qui comprennent un composé de formule (I), ou un sel d'addition de ce dernier à un acide pharmaceutiquement acceptable, ou encore un hydrate ou un solvate.

Ces médicaments trouvent leur emploi en thérapeutique, notamment dans le traitement et la prévention de la douleur aiguë ou chronique notamment périphériques ou associées à des maladies inflammatoires telles que l'arthrite, l'arthrite rhumatoïde, l'ostéoarthrite, la spondylite, la goutte, la vascularite, la maladie de Crohn et le syndrome du colon irritable, de douleurs neuropathiques, musculaires, osseuses, post-opératoires, de la migraine, les lombalgies, les douleurs associées aux cancers, au diabète au bien encore au SIDA.

Les composés selon l'invention trouvent encore leur emploi dans le traitement des dysfonctions sexuelles et en particulier dans le traitement de l'éjaculation précoce chez l'homme pour lesquelles l'activité de composé de type antalgique a été montrée.

Selon un autre de ses aspects, la présente invention concerne des compositions pharmaceutiques comprenant, en tant que principe actif, un composé selon l'invention. Ces compositions pharmaceutiques contiennent une dose efficace d'au moins un composé selon l'invention, ou un sel pharmaceutiquement acceptable, un hydrate ou solvat dudit composé, ainsi qu'au moins un excipient pharmaceutiquement acceptable.

Lesdits excipients sont choisis selon la forme pharmaceutique et le mode d'administration souhaité, parmi les excipients habituels qui sont connus de l'Homme du métier.

Dans les compositions pharmaceutiques de la présente invention pour l'administration orale, sublinguale, sous-cutanée, intramusculaire, intra-veineuse, topique, locale, intratrachéale, intranasale, transdermique ou rectale, intraoculaire, le principe actif de formule (I) ci-dessus, ou son sel, solvat ou hydrate éventuel peut être administré sous forme unitaire d'administration, en mélange avec des excipients pharmaceutiques classiques, aux animaux et aux êtres humains pour le traitement des troubles ou des maladies ci-dessus.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, transdermique, sous-cutanée, intramusculaire ou intraveineuse, les formes d'administration rectale et les implants. Pour l'application topique, on peut utiliser les composés selon l'invention dans des crèmes, gels, pommades ou lotions.

A titre d'exemple, une forme unitaire d'administration d'un composé selon l'invention sous forme de comprimé peut comprendre les composants suivants :

| | |
|---|---|
| Composé selon l'invention 1 | 50,0 mg |
| Mannitol | 223,75 mg |
| Croscarmellose sodique | 6,0 mg |
| Amidon de maïs | 15,0 mg |
| Hydroxypropyl-méthylcellulose | 2,25 mg |
| Stéarate de magnésium | 3,0 mg |

## Revendications

1. Composé de formule générale (I)

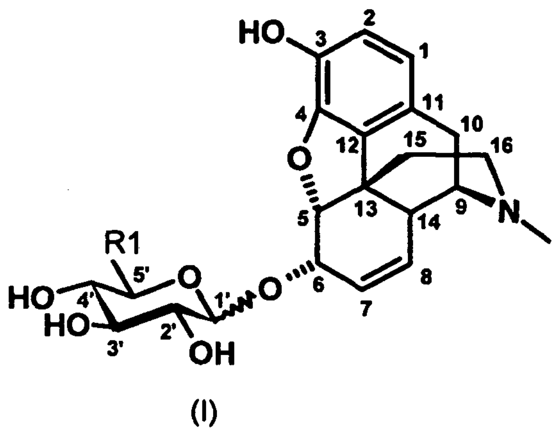

(I)

dans laquelle :

R1 est un groupe hétérocycle aromatique à 5 chaînons éventuellement substitué par un ou plusieurs substituants choisis parmi les atomes d'halogène et les groupes $(C_1-C_4)$alkyle, hydroxyle, oxo, halo$(C_1-C_4)$alkyle, halo$(C_1-C_4)$alkyloxy, $(C_1-C_4)$alkyloxy, aryl$(C_1-C_4)$alkyle et aryle, ledit groupe aryle étant éventuellement substitué par un ou plusieurs groupes choisis parmi les groupes $(C_1-C_4)$alkyle, halo$(C_1-C_4)$alkyle, hydroxyle et $(C_1-C_4)$alkyloxy,

à l'état de base ou de sel d'addition à un acide ainsi qu'à l'état d'hydrate ou de solvate.

**2.** Composé de formule générale (I) selon la revendication 1, **caractérisé en ce que** ledit composé présente une ou plusieurs des caractéristiques suivantes :

- le groupe hétérocycle aromatique est choisi parmi les groupes pyrrole, furane, thiophène, imidazole, triazole, tetrazole, oxazole, isoxazole, oxadiazole, thiazole, isothiazole et thiadiazole, et
- lorsque le groupe hétérocycle aromatique est substitué par un ou plusieurs groupes, ledit groupe est choisi parmi les groupes méthyle, éthyle, propyle, isopropyle, butyle, isobutyle, tertbutyle, fluorométhyle, difluorométhyle, trifluorométhyle, chlorométhyle, dichlorométhyle, trichlorométhyle, fluoroéthyle, difluoroéthyle, trifluoroéthyle, chloroéthyle, dichloroéthyle, trichloroéthyle, méthoxyphényle, éthoxyphényle, propoxyphényle et butyloxyphényle.

**3.** Composé de formule générale (I) selon la revendication 1, **caractérisé en ce que** :

- ledit groupe hétérocycle aromatique est choisi parmi les groupes tétrazole, triazole et oxadiazole, et
- lorsque ledit groupe hétérocycle aromatique est substitué par un ou plusieurs groupes, ledit groupe est choisi parmi les groupes méthyle, trifluoroéthyle et p-méthoxyphényle.

**4.** Composé de formule générale (I) selon l'une quelconque des revendications précédentes, **caractérisé en ce qu'**il est choisi parmi :

- le morphin-6-yl 5-C-(tétrazol-5-yl)-α/β-D-xylopyranoside (1/1),
- le morphin-6-yl 5-C-(tétrazol-5-yl)-β-D-xylopyranoside
- le morphin-6-yl 5-C-(2-méthyl-1,3,4-oxadiazol-5-yl)- α/β-D-xylopyranoside (2/3)
- le morphin-6-yl 5-C-(2-méthyl-1,3,4-oxadiazol-5-yl)- α-D-xylopyranoside
- le morphin-6-yl 5-C-(2-méthyl-1,3,4-oxadiazol-5-yl)-β-D-xylopyranoside et
- le morphin-6-yl 5-C-[5-(4-méthoxyphényl)-4-(2,2,2-trifluoroéthyl)-4H-1,2,4-triazol-3-yl]-β-D-xylopyranoside.

**5.** Médicament, **caractérisé en ce qu'**il comprend un composé de formule générale (I) selon l'une quelconque des revendications 1 à 4, ou un sel d'addition de ce composé à un acide pharmaceutiquement acceptable, ou encore un hydrate ou un solvate du composé de formule (I).

**6.** Composition pharmaceutique, **caractérisée en ce qu'**elle comprend un composé de formule générale (I) selon l'une quelconque des revendications 1 à 4, ou un sel pharmaceutiquement acceptable, un hydrate ou un solvate de ce composé, ainsi qu'au moins un excipient pharmaceutiquement acceptable.

**7.** Utilisation d'un composé de formule générale (I) selon l'une quelconque des revendications 1 à 4 pour la préparation d'un médicament destiné au traitement ou à la prévention de la douleur.

**8.** Composé de formule générale (III)

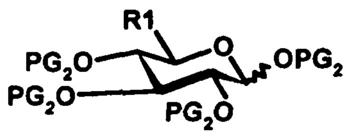

**(III)**

dans laquelle :

- R1 est tel que défini en revendication 1, et
- $PG_2$ représente un groupe benzoyle.

**9.** Composé de formule générale (III) selon la revendication 8, **caractérisé en ce que** R1 est choisi parmi les groupes tétrazole, 1,2,3,4-tétra-*O*-benzoyl-5-*C*-2-(4-méthoxybenzyl)-2H-tétrazole, 1,2,3,4-tétra-*O*-benzoyl-5-C-1-(4-méthoxybenzyl)-1*H*-tétrazole et 5(4-méthoxyphényl) -4-(2,2,2-trifluoroéthyle) 1,2,4 triazole.

**10.** Composé de formule générale (IV)

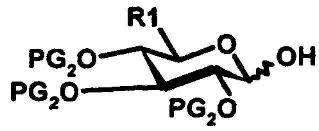

**(IV)**

dans laquelle :

- R1 est tel que défini en revendication 1, et
- $PG_2$ représente un groupe benzoyle.

**11.** Composé de formule générale (IV) selon la revendication 10, **caractérisé en ce que** R1 est choisi parmi les groupes 1,2,3,4-tétra-*O*-benzoyl-5-*C*-2-(4-méthoxybenzyl)-2*H*-tétrazole, 1,2,3,4-tétra-*O*-benzoyl-5-*C*-1-(4-méthoxybenzyl)-1*H*-tétrazole et 5(4-méthoxyphényl) -4-(2,2,2-trifluoroéthyle) 1,2,4 triazole.

**12.** Composé de formule générale (V)

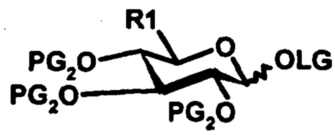

**(V)**

dans laquelle :

- R1 est tel que défini en revendication 1
- $PG_2$ représente un groupe benzoyle, et
- LG représente un groupe trichloroacétamidate.

**13.** Composé de formule générale (V) selon la revendication 12, **caractérisé en ce que** R1 est choisi parmi les groupes 1,2,3,4-tétra-*O*-benzoyl-5-*C*-2-(4-méthoxybenzyl)-2*H*-tétrazole, 1,2,3,4-tétra-*O*-benzoyl-5-*C*-1-(4-méthoxybenzyl)-1*H*-tétrazole et 5(4-méthoxyphényl) -4-(2,2,2-trifluoroéthyle) 1,2,4 triazole.

**Patentansprüche**

**1.** Verbindung der allgemeinen Formel (I)

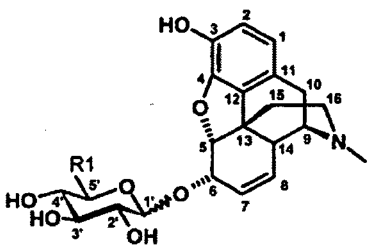

(I)

worin:

R1 für eine 5-gliedrige aromatische heterocyclische Gruppe steht, die gegebenenfalls durch einen oder mehrere Substituenten, die aus Halogenatomen und ($C_1$-$C_9$)-Alkyl-, Hydroxyl-, Oxo-, Halogen-($C_1$-$C_4$)-alkyl-, Halogen-($C_1$-$C_4$)-alkyloxy-, ($C_1$-$C_4$) -Alkyloxy-, Aryl- ($C_1$-$C_4$)-alkyl- und Arylgruppen ausgewählt sind, substituiert ist, wobei die Arylgruppe gegebenenfalls durch eine oder mehrere Gruppen, die aus($C_1$-$C_4$)-Alkyl-, Halogen-($C_1$-$C_4$-alkyl-, Hydroxyl- und ($C_1$-$C_4$)-Alkyloxy-gruppen ausgewählt sind, substituiert ist, in Basen- oder Säureadditionssalzform sowie in Hydrat- oder Solvatform.

2. Verbindung der allgemeinen Formel (I) nach Anspruch 1, **dadurch gekennzeichnet, dass** die Verbindung eines oder mehrere der folgenden Merkmale aufweist:

- die aromatische heterocyclische Gruppe ist aus Pyrrol-, Furan-, Thiophen-, Imidazol-, Triazol-, Tetrazol-, Oxazol-, Isoxazol-, Oxadiazol-, Thiazol-, Isothiazol- und Thiadiazolgruppen ausgewählt und
- dann, wenn die aromatische heterocyclische Gruppe durch eine oder mehrere Gruppen substituiert ist, die Gruppe aus Methyl-, Ethyl-, Propyl-, Isopropyl-, Butyl-, Isobutyl-, tert.-Butyl-, Fluormethyl-, Difluormethyl-, Trifluormethyl-, Chlormethyl-, Dichlormethyl-, Trichlormethyl-, Fluorethyl-, Difluorethyl-, Trifluorethyl-, Chlorethyl-, Dichlorethyl-, Trichlorethyl-, Methoxyphenyl-, Ethoxyphenyl-, Propoxyphenyl- und Butyloxyphenylgruppen ausgewählt ist.

3. Verbindung der allgemeinen Formel (I) nach Anspruch 1, **dadurch gekennzeichnet, dass**:

- die aromatische heterocyclische Gruppe aus Tetrazol-, Triazol- und Oxadiazolgruppen ausgewählt ist und
- dann, wenn die aromatische heterocyclische Gruppe durch eine oder mehrere Gruppen substituiert ist, die Gruppe aus Methyl-, Trifluormethyl- und p-Methoxyphenylgruppen ausgewählt ist.

4. Verbindung der allgemeinen Formel (I) nach einem der vorhergehenden Ansprüche, **dadurch gekennzeichnet, dass** sie ausgewählt ist aus:

- Morphin-6-yl-5-C-(tetrazol-5-yl)-α/β-D-xylopyranosid (1/1)
- Morphin-6-yl-5-C-(tetrazol-5-yl)-β-D-xylopyranosid
- Morphin-6-yl-5-C-(2-methyl-1,3,4-oxadiazol-5-yl)-α/β-D-xylopyranosid (2/3)
- Morphin-6-yl-5-C-(2-methyl-1,3,4-oxadiazol-5-yl)-α-D-xylopyranosid
- Morphin-6-yl-5-C-(2-methyl-1,3,4-oxadiazol-5-yl)-β-D-xylopyranosid und
- Morphin-6-yl-5-C-[5-(4-methoxyphenyl)-4-(2,2,2-trifluorethyl)-4H-1,2,4-triazo1-3-yl]-β-D-xylopyranosid.

5. Medikament, **dadurch gekennzeichnet, dass** es eine Verbindung der allgemeinen Formel (I) nach einem der Ansprüche 1 bis 4 oder ein Additionssalz dieser Verbindung mit einer pharmazeutisch unbedenklichen Säure oder auch ein Hydrat oder ein Solvat der Verbindung der Formel (I) umfasst.

6. Pharmazeutische Zusammensetzung, **dadurch gekennzeichnet, dass** sie eine Verbindung der allgemeinen Formel (I) nach einem der Ansprüche 1 bis 4 oder ein pharmazeutisch unbedenkliches Salz, ein Hydrat oder ein Solvat dieser Verbindung sowie mindestens einen pharmazeutisch unbedenklichen Hilfsstoff umfasst.

7. Verwendung einer Verbindung der allgemeinen Formel (I) nach einem der Ansprüche 1 bis 4 zur Herstellung eines Medikaments zur Behandlung oder Prävention von Schmerzen.

8. Verbindung der allgemeinen Formel (III)

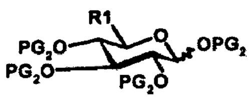

(III)

worin:

- R1 wie in Anspruch 1 definiert ist und
- $PG_2$ für eine Benzoylgruppe steht.

**9.** Verbindung der allgemeinen Formel (III) nach Anspruch 8, **dadurch gekennzeichnet, dass** R1 aus Tetrazol-, 1,2,3,4-Tetra-*O*-benzoyl-5-*C*-2-(4-methoxybenzyl)-2*H*-tetrazol-, 1,2,3,4-Tetra-*O*-benzoyl-5-*C*-1-(4-methoxybenzyl)-1*H*-tetrazol- und 5-(4-Methoxyphenyl)-4-(2,2,2-trifluorethyl)-1,2,4-triazolgruppen ausgewählt ist.

**10.** Verbindung der allgemeinen Formel (IV)

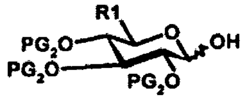

**(IV)**

worin:

- R1 wie in Anspruch 1 definiert ist und
- $PG_2$ für eine Benzoylgruppe steht.

**11.** Verbindung der allgemeinen Formel (IV) nach Anspruch 10, **dadurch gekennzeichnet, dass** R1 aus 1,2,3,4-Tetra-*O*-benzoyl-5-*C*-2-(4-methoxybenzyl)-2H-tetrazol-, 1,2,3,4-Tetra-*O*-benzoyl-5-*C*-1-(4-methoxybenzyl)-1*H*-tetrazol- und 5-(4-Methoxyphenyl)-4-(2,2,2-trifluorethyl)-1,2,4-triazolgruppen ausgewählt ist.

**12.** Verbindung der allgemeinen Formel (V)

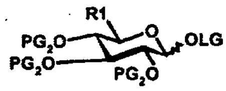

**(V)**

worin:

- R1 wie in Anspruch 1 definiert ist,
- $PG_2$ für eine Benzoylgruppe steht und
- LG für eine Trichloracetamidatgruppe steht.

**13.** Verbindung der allgemeinen Formel (V) nach Anspruch 12, **dadurch gekennzeichnet, dass** R1 aus 1,2,3,4-Tetra-*O*-benzoyl-5-*C*-2-(4-methoxybenzyl)-2*H*-tetrazol-, 1,2,3,4-Tetra-*O*-benzoyl-5-*C*-1-(4-methoxybenzyl)-1*H*-tetrazol- und 5-(4-Methoxyphenyl)-4-(2,2,2-trifluorethyl)-1,2,4-triazolgruppen ausgewählt ist.

## Claims

**1.** Compound of general formula (I)

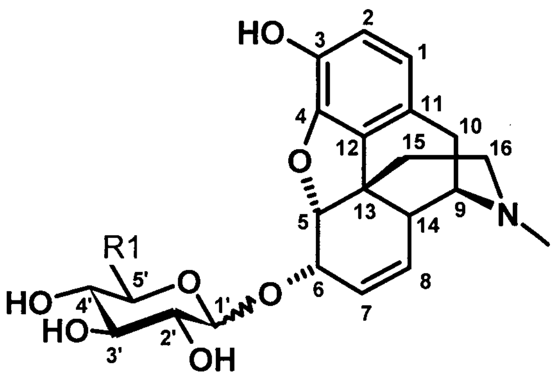

(I)

in which:

R1 is a 5-membered aromatic heterocyclic group optionally substituted with one or more substituents chosen from halogen atoms and groups ($C_1$-$C_4$) alkyl, hydroxyl, oxo, halo ($C_1$-$C_4$) alkyl, halo ($C_1$-$C_4$) alkyloxy, ($C_1$-$C_9$) alkyloxy, aryl($C_1$-$C_4$)alkyl and aryl, the said aryl group being optionally substituted with one or more groups chosen from the groups ($C_1$-$C_4$)alkyl, halo ($C_1$-$C_4$)alkyl, hydroxyl and ($C_1$-$C_4$)alkyloxy,
in the form of base or of an acid-addition salt, and also in the form of a hydrate or a solvate.

**2.** Compound of general formula (I) according to Claim 1, **characterized in that** the said compound has one or more of the following characteristics:

- the aromatic heterocyclic group is chosen from pyrrole, furan, thiophene, imidazole, triazole, tetrazole, oxazole, isoxazole, oxadiazole, thiazole, isothiazole and thiadiazole groups, and
- when the aromatic heterocyclic group is substituted with one or more groups, the said group is chosen from methyl, ethyl, propyl, isopropyl, butyl, isobutyl, tert-butyl, fluoromethyl, difluoromethyl, trifluoromethyl, chloromethyl, dichloromethyl, trichloromethyl, fluoroethyl, difluoroethyl, trifluoroethyl, chloroethyl, dichloroethyl, trichloroethyl, methoxyphenyl, ethoxyphenyl, propoxyphenyl and butyloxyphenyl groups.

**3.** Compound of general formula (I) according to Claim 1, **characterized in that**:

- the said aromatic heterocyclic group is chosen from tetrazole, triazole and oxadiazole groups, and
- when the said aromatic heterocyclic group is substituted with one or more groups, the said group is chosen from methyl, trifluoroethyl and p-methoxyphenyl groups.

**4.** Compound of general formula (I) according to any one of the preceding claims, **characterized in that** it is chosen from:

- morphin-6-yl 5-C-(tetrazol-5-yl)-α/β-D-xylopyranoside (1/1)
- morphin-6-yl 5-C-(tetrazol-5-yl)-β-D-xylopyranoside
- morphin-6-yl 5-C-(2-methyl-1,3,4-oxadiazol-5-yl)- α/β-D-xylopyranoside (2/3)
- morphin-6-yl 5-C-(2-methyl-1,3,4-oxadiazol-5-yl)- α -D-xylopyranoside
- morphin-6-yl 5-C-(2-methyl-1,3,4-oxadiazol-5-yl)-β-D-xylopyranoside and
- morphin-6-yl 5-C-[5-(4-methoxyphenyl)-4-(2,2,2-trifluoroethyl)-4H-1,2,4-triazol-3-yl]-β-D-xylopyranoside.

**5.** Medicament, **characterized in that** it comprises a compound of general formula (I) according to any one of Claims 1 to 4, or an addition salt of this compound with a pharmaceutically acceptable acid, or a hydrate or solvate of the compound of formula (I).

**6.** Pharmaceutical composition, **characterized in that** it comprises a compound of general formula (I) according to any one of Claims 1 to 4, or a pharmaceutically acceptable solvent, hydrate or solvate of this compound, and also at least one pharmaceutically acceptable excipient.

**7.** Use of a compound of general formula (I) according to any one of Claims 1 to 4, for the preparation of a medicament for treating or preventing pain.

**8.** Compound of general formula (III)

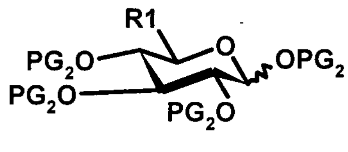

**(III)**

in which:

- R1 is as defined in Claim 1, and
- $PG_2$ represents a benzoyl group.

**9.** Compound of general formula (III) according to Claim 8, **characterized in that** R1 is chosen from tetrazole, 1,2,3,4-tetra-*O*-benzoyl-5-*C*-2-(4-methoxybenzyl)-2*H*-tetrazole, 1,2,3,4-tetra-*O*-benzoyl-5-*C*-1-(4-methoxybenzyl)-1H-tetrazole and 5-(4-methoxyphenyl)-4-(2,2,2-trifluoroethyl)-1,2,4-triazole groups.

**10.** Compound of general formula (IV)

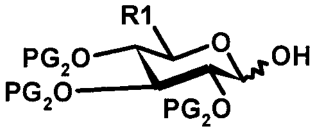

**(IV)**

in which:

- R1 is as defined in Claim 1, and
- $PG_2$ represents a benzoyl group.

**11.** Compound of general formula (IV) according to Claim 10, **characterized in that** R1 is chosen from 1,2,3,4-tetra-*O*-benzoyl-5-*C*-2-(4-methoxybenzyl)-2*H*-tetrazole, 1,2,3,4-tetra-*O*-benzoyl-5-*C*-1-(4-methoxybenzyl)-1*H*-tetrazole and 5-(4-methoxyphenyl)-4-(2,2,2-trifluoroethyl)-1,2,4-triazole groups.

**12.** Compound of general formula (V)

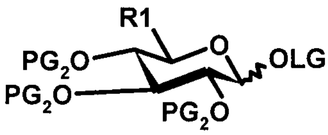

**(V)**

in which:

- R1 is as defined in Claim 1,
- $PG_2$ represents a benzoyl group, and
- LG represents a trichloroacetamidate group.

**13.** **Compound** of general formula (V) according to Claim 12, **characterized in that** R1 is chosen from 1,2,3,4-tetra-*O*-benzoyl-5-*C*-2-(4-methoxybenzyl)-2*H*-tetrazole, 1,2,3,4-tetra-*O*-benzoyl-5-*C*-1-(4-methoxybenzyl)-1*H*-tetrazole and 5-(4-methoxyphenyl)-4-(2,2,2-trifluoroethyl)-1,2,4-triazole groups.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 9846618 A
- FR 2864082


### Littérature non-brevet citée dans la description


- **GREEN et al.** Protective Groups in Organic Synthesis. John Wiley & Sons, Inc,
- **J. MARCH.** Advances in Organic Chemistry. Wiley Interscience, 310-316
- *Portoghese and coll. J. Med. Chem,* 1972, vol. 15, 208-210
- *Carbohydrate Research,* 2006, vol. 341 (1), 41-48
- *Tetrahedron,* 2000, vol. 56, 7591-7594